# EUROPEAN PATENT SPECIFICATION

(11) **EP 2 585 112 B1**
(45) Date of publication and mention of the grant of the patent: **15.05.2019**
(21) Application number: 11798931.9
(22) Date of filing: 23.06.2011
(51) Int. Cl.: A61K 45/00, A61P 9/00, A61K 38/14, A61K 31/726, A61K 31/727

(54) **COLLAGEN-BINDING SYNTHETIC PEPTIDOGLYCANS FOR USE IN VASCULAR INTERVENTION**
KOLLAGENBINDENDE SYNTHETISCHE PEPTIDOGLYCANE ZUR VERWENDUNG BEI GEFÄSSEINGRIFFEN
PEPTIDOGLYCANES SYNTHÉTIQUES SE LIANT AU COLLAGÈNE DESTINÉS À ÊTRE UTILISÉS DANS DES OPÉRATIONS VASCULAIRES

(30) Priority: 23.06.2010 US 357912 P
(43) Date of publication of application: 01.05.2013
(73) Proprietor: Purdue Research Foundation, West Lafayette, IN 47906 (US)
(72) Inventor: PADERI, John, E., Lafayette, IN 47901 (US); PANITCH, Alyssa, West Lafayette, IN 47906 (US); PARK, Kinam, West Lafayette, IN 47906 (US); STUART, Katherine, Lafayette, IN 47901 (US)
(74) Representative: Wise, Daniel Joseph
(86) International application number: PCT/US2011/041654
(87) International publication number: WO 2011/163492

(56) References cited:
- WO-A2-2009/120995
- US-A1- 2004 236 092
- US-A1- 2005 113 297
- US-A1- 2007 167 441
- US-A1- 2008 131 466
- US-A1- 2010 111 842
- US-A1- 2011 020 298
- Nafiseh Nili ET AL: "Decorin inhibition of PDGF-stimulated vascular smooth muscle cell function: potential mechanism for inhibition of intimal hyperplasia after balloon angioplasty", The American journal of pathology, 1 September 2003 (2003-09-01), pages 869-878, XP055090848, United States Retrieved from the Internet: URL:http://www.pubmedcentral.nih.gov/artic lerender.fcgi?artid=1868258&tool=pmcentrez &rendertype=abstract [retrieved on 2013-11-29]
- REBECCA A. SCOTT ET AL: "Decorin Mimic Inhibits Vascular Smooth Muscle Proliferation and Migration", PLOS ONE, vol. 8, no. 11, 22 November 2013 (2013-11-22), page e82456, XP055090845, DOI: 10.1371/journal.pone.0082456
- PADERI JOHN E ET AL: "The inhibition of platelet adhesion and activation on collagen during balloon angioplasty by collagen-binding peptidoglycans.", BIOMATERIALS APR 2011, vol. 32, no. 10, April 2011 (2011-04), pages 2516-2523, XP0028133675, ISSN: 1878-5905
- KATE STUART ET AL: "Collagen-Binding Peptidoglycans Inhibit MMP Mediated Collagen Degradation and Reduce Dermal Scarring", PLOS ONE, vol. 6, no. 7, 11 June 2011 (2011-06-11), page e22139, XP055090780, ISSN: 1932-6203, DOI: 10.1371/journal.pone.0022139
- PADERI ET AL.: 'Collagen-Binding Peptidoglycans: A Biomimetic Approach to Modulate Collagen Fibrillogenesis for Tissue Engineering Applications' TISSUE ENGINEERING PART A vol. 15, no. 10, 2009, pages 2991 - 2999, XP003030684
- GRIESE ET AL.: 'Isolation and Transplantation of Autologous Circulating Endothelial Cells Into Denuded Vessels and Prosthetic Grafts: Implications for Cell-Based Vascular Therapy' CIRCULATION vol. 108, no. 21, 2003, pages 2710 - 2715, XP003021959
- CHIANG ET AL.: 'A synthetic peptide derived from the sequence of a type I collagen receptor inhibits type collagen-mediated platelet aggregation' J CLIN INVEST vol. 100, no. 8, 1997, pages 2079 - 2084, XP002118050
- WANG ET AL.: 'Platelet, not endothelial, p-selectin is required for neointimal formation after vascular injury' ARTERIOSCLER THROMB VASC BIOL vol. 25, no. 8, 2005, pages 1584 - 1589, XP003030685
- RATCLIFFE: 'Tissue engineering of vascular grafts' MATRIX BIOLOGY vol. 19, 2000, pages 353 - 357, XP002977518
- CHUPA ET AL.: 'Vascular cell responses to polysaccharide materials: in vitro and in vivo evaluations' BIOMATERIALS vol. 21, no. 22, 2000, pages 2315 - 2322, XP004210289
- PADERI ET AL.: 'Design of a Synthetic Collagen-Binding Peptidoglycan that Modulates Collagen Fibrillogenesis' BIOMACROMOLECULES vol. 9, 2008, pages 2562 - 2566, XP002688096
- PADERI ET AL.: 'The inhibition of platelet adhesion and activation on collagen during balloon angioplasty by collagen-binding peptidoglycans' BIOMATERIALS vol. 32, 2011, pages 2516 - 2523, XP028133675
- PIEPER ET AL.: 'Development of tailor-made collagen}glycosaminoglycan matrices: EDC/NHS crosslinking, and ultrastructural aspects' BIOMATERIALS vol. 21, 2000, pages 581 - 593, XP004187176
- MAMMEN ET AL.: 'Polyvalent Interactions in Biological Systems: Implications for Design and Use of Multivalent Ligands and Inhibitors' ANGEW. CHEM. INT. ED. vol. 37, 1998, pages 2754 - 2794, XP002103106
- LEE J Y ET AL: "Injectable gel with synthetic collagen-binding peptide for enhanced osteogenesis in vitro and in vivo", BIOCHEMICAL AND BIOPHYSICAL RESEARCH COMMUNICATIONS, ACADEMIC PRESS INC. ORLANDO, FL, US, vol. 357, no. 1, 25 May 2007 (2007-05-25), pages 68-74, XP026863460, ISSN: 0006-291X, DOI: 10.1016/J.BBRC.2007.03.106 [retrieved on 2007-04-18]

## Description

### TECHNICAL FIELD

This invention pertains to the field of collagen-binding synthetic peptidoglycans. More particularly, this invention relates to collagen-binding synthetic peptidoglycans for use in vascular intervention procedures.

### BACKGROUND AND SUMMARY OF THE INVENTION

Vascular interventions that involve a medical device inserted or implanted into the body of a patient, for example, angioplasty, stenting, atherectomy and grafting, are often associated with undesirable effects. For example, the insertion or implantation of catheters or stents can lead to the formation of emboli or clots in blood vessels. Other adverse reactions to vascular intervention can include hyperplasia, restenosis, occlusion of blood vessels, platelet aggregation, and calcification.

The number of percutaneous coronary intervention (PCI) procedures, commonly known as balloon angioplasty, has increased by 30% over the past 10 years totaling more than 1.3 million patients in the U.S. annually at a cost of more than $60 billion. During percutaneous coronary intervention, guide catheters are advanced from the periphery, usually the femoral artery, into the aorta. The tip of the catheter is positioned in the ostium of a coronary artery. Subsequently, wires, balloon catheters, or other devices are advanced through the guide catheter into the large epicardial coronary arteries to treat stenotic lesions.

The large increase in the number of procedures is due to a rise in heart disease as well as technological advancements, which has led to safer and more effective practice. Still, PCI procedures are not without problems including thrombosis and intimal hyperplasia, which are complications from the procedure. Areas of focus for mitigating these complications are the coagulation and inflammatory responses which occur at the vessel wall as a result of the procedure. Balloon inflation results in endothelial denudation of the vessel wall, which initiates coagulation and inflammation through platelet activation and is currently a possible consequences of PCI procedures.

WO2009/120995A2 relates to collagen-binding synthetic peptidoglycans and engineered collagen matrices comprising a collagen matrix and a collagen-binding synthetic peptidoglycan where the collagen-binding synthetic peptidoglycan.

Nili et al., "Decorin Inhibition of PDGF-stimulated vascular smooth muscle cell function", The American Journal of Pathology, September 2003, pages 869-878.

Paderi et al. "Collagen-Binding Peptidoglycans: A Biomimetic Approach to Modulate Collagen Fibrillogenesis for Tissue Engineering Applications", Tissue Engineering Part A, 2009, Vol. 15, No. 10, pages 2991-2999.

Paderi et al. "Design of a Synthetic Collagen-Binding Peptidoglycan that Modulates Collagen Fibrillogenesis", Biomacromolecules, 2008, Vol. 9, pages 2562-2566.

Lee et al. "Injectable gel with synthetic collagen-binding peptide for enhanced osteogenesis in vitro and in vivo", Biochemical and Biophysical Research Communications, Academic Press Inc., May 2007, Vol. 357, No.1, pages 68-74.

The synthetic collagen-binding peptidoglycans described herein can be synthesized with design control and in large quantities at low cost, making their clinical use feasible. As described herein the synthetic collagen-binding peptidoglycans are designed to bind collagen with high affinity, where they remain bound during blood flow to prevent platelet binding to exposed collagen of the denuded endothelium and, consequently, to prevent platelet activation, thrombosis, inflammation resulting from denuding the endothelium, intimal hyperplasia, and vasospasm. The collagen-binding synthetic peptidoglycans described herein can also stimulate endothelial cell proliferation and can bind to collagen in a denuded vessel.

The present invention relates to a collagen binding peptidoglycan of formula (PₙL)ₓG, for use in a method for treating i) vascular injury or ii) intimal hyperplasia according to any of claims 1 to 9.

The following numbered embodiments are contemplated:
1. A collagen binding peptidoglycan according to any of claims 1 to 9 for use in vascular intervention in a patient, wherein the collagen-binding synthetic peptidoglycan binds to a denuded vessel in the patient.
2. The collagen binding peptidoglycan for use according to embodiment 1 wherein the collagen-binding synthetic peptidoglycan inhibits platelet activation.
3. The collagen binding peptidoglycan for use according to any of embodiments 1 to 2 wherein the collagen binding synthetic peptidoglycan inhibits platelet binding to the denuded vessel.
4. The collagen binding peptidoglycan for use according to any of embodiments 1 to 3 wherein the collagen binding synthetic peptidoglycan inhibits intimal hyperplasia.
5. The collagen binding peptidoglycan for use according to any of embodiments 1 to 4 wherein the collagen binding synthetic peptidoglycan inhibits inflammation resulting from denuding of the vessel.
6. The collagen binding peptidoglycan for use according to any of embodiments 1 to 5 wherein the collagen binding synthetic peptidoglycan inhibits thrombosis.
7. The collagen binding peptidoglycan for use according to any of embodiments 1 to 6 wherein the collagen binding synthetic peptidoglycan inhibits vasospasm.
8. The collagen binding peptidoglycan for use according to any of embodiments 1 to 7 wherein the collagen binding synthetic peptidoglycan stimulates endothelial cell proliferation.
9. The collagen binding peptidoglycan for use according to any of embodiments 1 to 8 wherein the collagen binding synthetic peptidoglycan binds to exposed collagen on the denuded vessel.
10. The collagen binding peptidoglycan for use according to any of embodiments 1 to 9 wherein the glycan is a glycosaminoglycan or a polysaccharide.
11. The collagen binding peptidoglycan for use according to any of embodiments 1 to 10 wherein the collagen binding synthetic peptidoglycan is DS-SILY₁₈.
12. The collagen binding peptidoglycan for use according to any of embodiments 1 to 11 wherein the collagen binding synthetic peptidoglycan is administered to the patient parenterally.
13. The collagen binding peptidoglycan for use according to embodiment 12 wherein the parenteral administration is through a route selected from the group consisting of intravascular, intravenous, intraarterial, intramuscular, cutaneous, subcutaneous, percutaneous, intradermal, and intraepidermal.
14. The collagen binding peptidoglycan for use according to any of embodiments 12 or 13 wherein the collagen binding synthetic peptidoglycan is administered parenterally using a needle or a device for infusion.
15. The collagen binding peptidoglycan for use according to any of embodiments 1 to 14 wherein the collagen binding synthetic peptidoglycan is administered to the patient with a catheter, as a coating on a ballon, through a porous ballon, or as a coating on a stent.
16. A kit comprising

   a collagen-binding synthetic peptidoglycan for use according to claims 1 to 9; and
   a component selected from the group consisting of a catheter, a stent, a balloon, and a combination thereof.

### BRIEF DESCRIPTION OF THE DRAWINGS

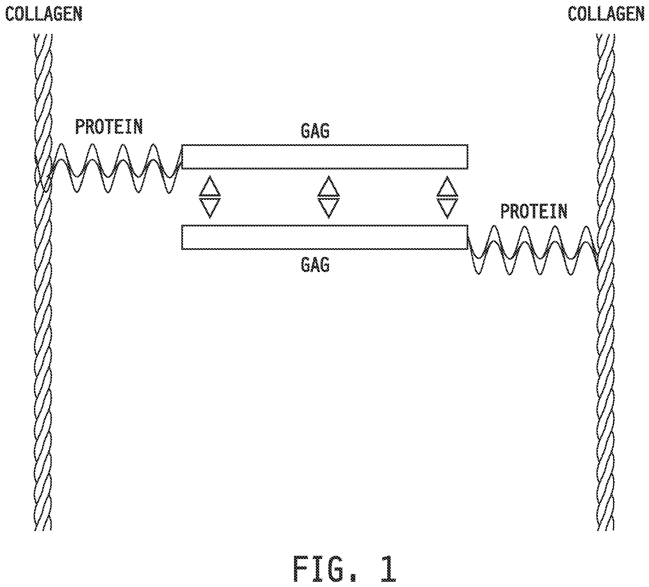

FIGURE 1 shows a schematic representation of the interaction between neighboring proteoglycans on adjacent tropocollagen strands which is important in determining the mechanical and alignment properties of collagen matrices.

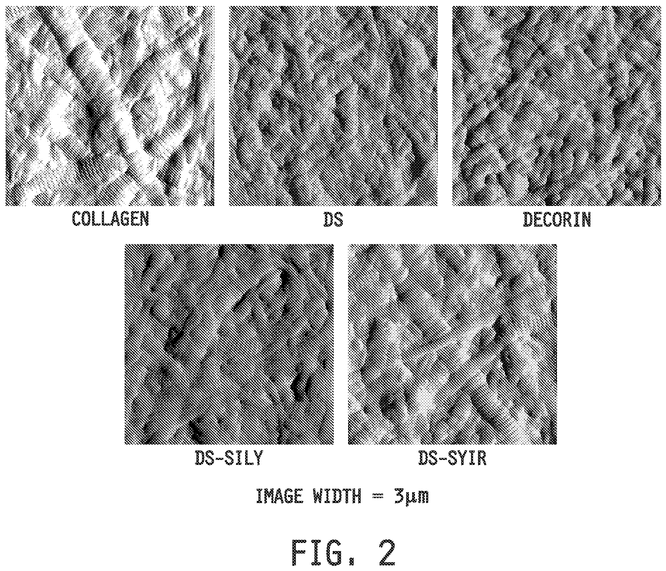

FIGURE 2. AFM images made in contact mode, with a scan rate of 2 Hz with Silicon Nitride contact mode tip k=0.05N/m tips and deflection setpoint: 0-1 Volts, of gel samples prepared as in EXAMPLE 15 (10:1 collagen:treatment) after dehydration with ethanol. Samples are for collagen alone (Collagen), and for collagen with dermatan sulfate (DS), with decorin (Decorin), dermatan sulfate-RRANAALKAGELYKSILYGC conjugate (DS-SILY) and dermatan sulfate- SYIRIADTNIT conjugate (DS-SYIR).

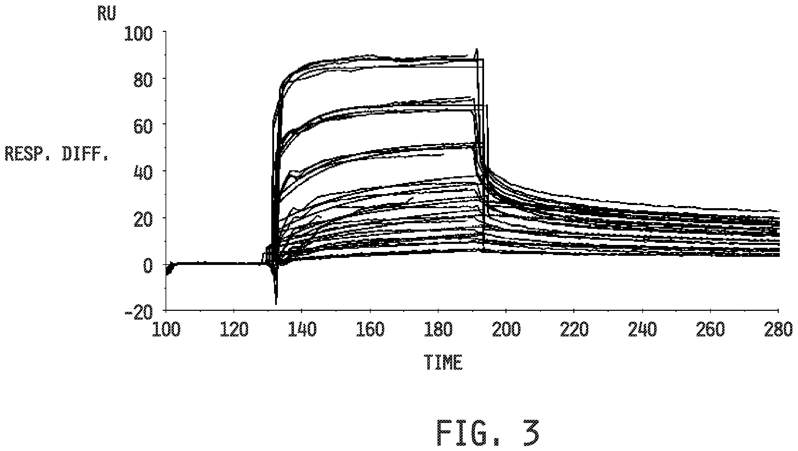

FIGURE 3. Surface Plasmon Resonance scan in association mode and dissociation mode of peptide RRANAALKAGELYKSILYGC (SILY) binding to collagen bound to CM-3 plates. SILY was dissolved in 1x HBS-EP buffer at varying concentrations from 100µM to 1.5µm in 2-fold dilutions.

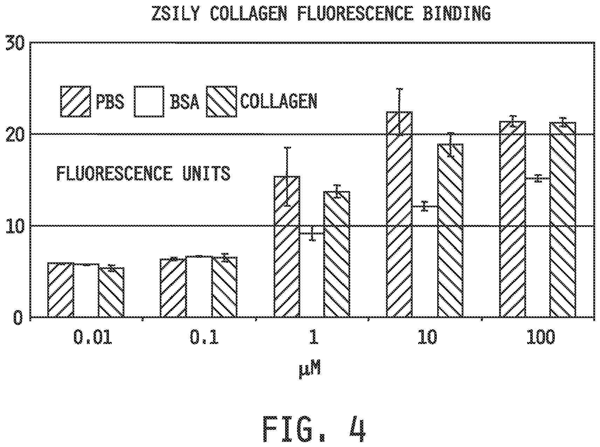

FIGURE 4. Binding of dansyl-modified peptide SILY to collagen measured in 96-well high-binding plate (black with a clear bottom (Costar)). PBS, buffer only; BSA, BSA-treated well; Collagen, collagen-treated well. Fluorescence readings were taken on an M5 Spectramax Spectrophotometer (Molecular Devices) at excitation/emission wavelengths of 335nm/490nm, respectively.

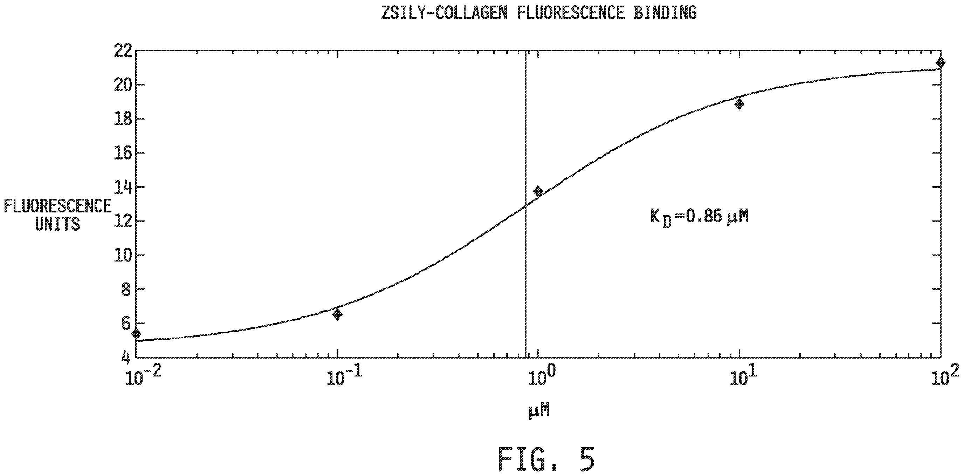

FIGURE 5. Collagen-dansyl-modified peptide SILY binding curve derived from fluorescence data described in FIGURE 4.

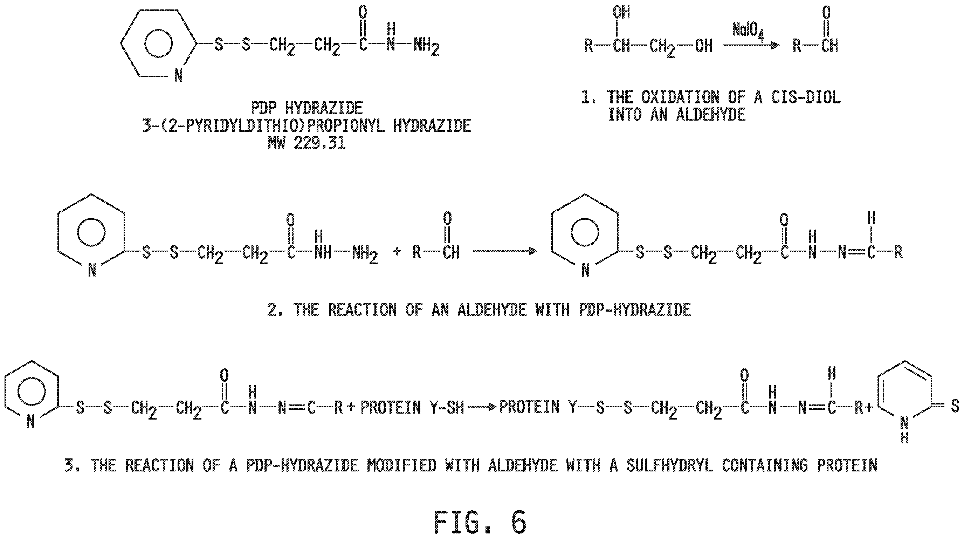

FIGURE 6. A schematic description of the reagent, PDPH, and the chemistry of the two-step conjugation of a cysteine-containing peptide with an oxidized glycosylaminoglycoside showing the release of 2-pyridylthiol in the final step.

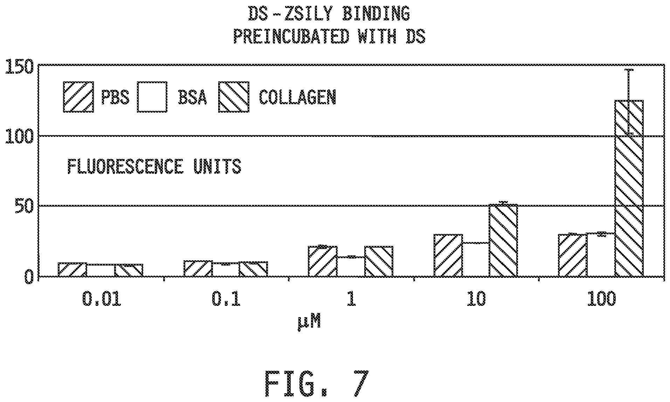

FIGURE 7. Binding of dansyl-modified peptide SILY conjugated to dermatan sulfate as described herein to collagen measured in 96-well high-binding plate (black with a clear bottom (Costar)). PBS, buffer only; BSA, BSA-treated well; Collagen, collagen-treated well. Fluorescence readings were taken on an M5 Spectramax Spectrophotometer (Molecular Devices) at excitation/emission wavelengths of 335nm/490nm respectively.

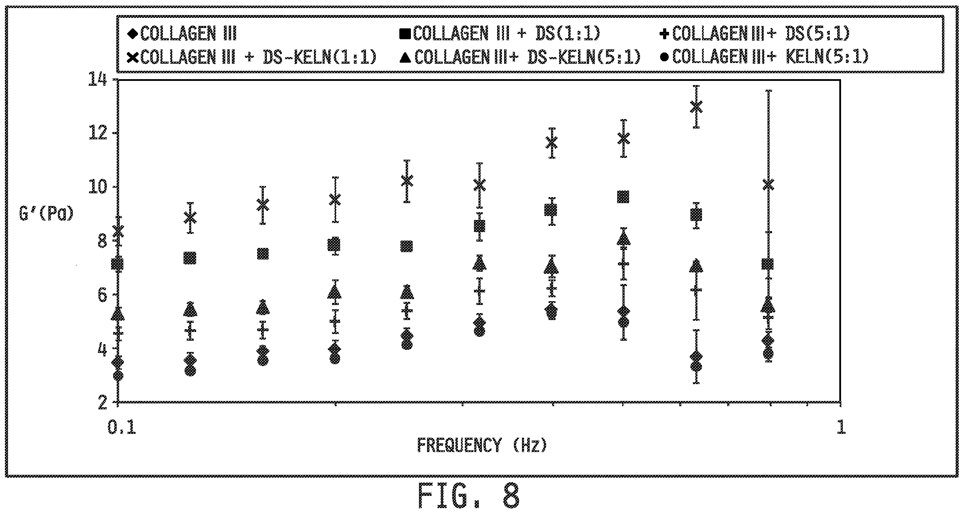

FIGURE 8. Measurement of Shear modulus of gel samples (1.5mg/mL collagen III, 5:1 collagen:treatment) on a AR-G2 rheometer with 20mm stainless steel parallel plate geometry (TA Instruments, New Castle, DE), and the 20mm stainless steel parallel plate geometry was lowered to a gap distance of 500µm using a normal force control of 0.25N. ◆ - no treatment, i.e. collagen III alone; ■ - collagen + dermatan sulfate (1:1); + - collagen + dermatan sulfate (5:1); x - collagen + dermatan sulfate-KELNLVYTGC (DS-KELN) conjugate (1:1); A - collagen + dermatan sulfate-KELN conjugate (5:1); ● - collagen + KELNLVYTGC (KELN) peptide.

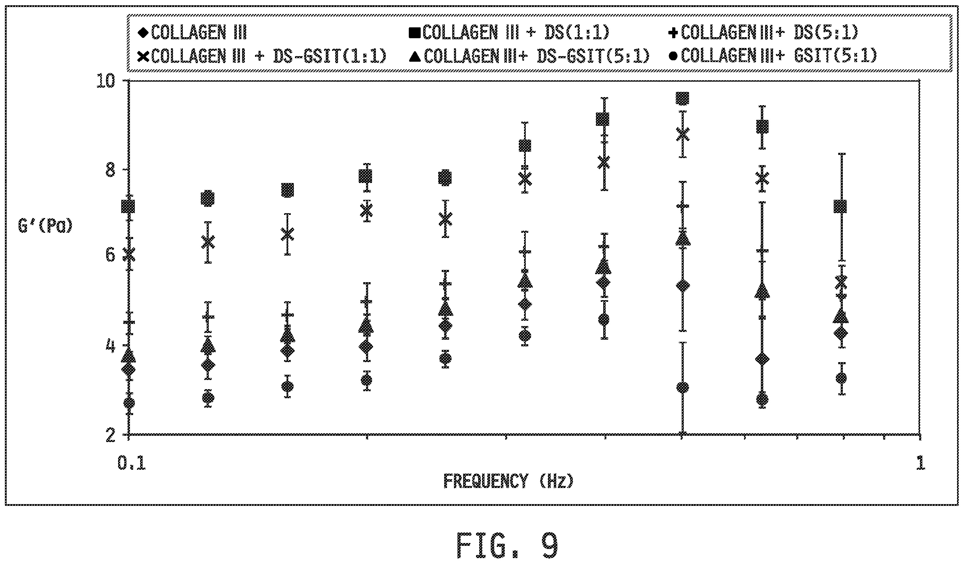

FIGURE 9. Measurement of Shear modulus of gel samples (1.5mg/mL collagen III, 5:1 collagen:treatment) on a AR-G2 rheometer with 20mm stainless steel parallel plate geometry (TA Instruments, New Castle, DE), and the 20mm stainless steel parallel plate geometry was lowered to a gap distance of 500µm using a normal force control of 0.25N. ◆ - no treatment, i.e. collagen III alone; ■ - collagen + dermatan sulfate (1:1); + - collagen + dermatan sulfate (5:1); **x** - collagen + dermatan sulfate-GSIT conjugate (DS-GSIT) (1:1); ▲ - collagen + dermatan sulfate-GSIT conjugate (5:1); ● - collagen + GSITTIDVPWNVGC (GSIT) peptide.

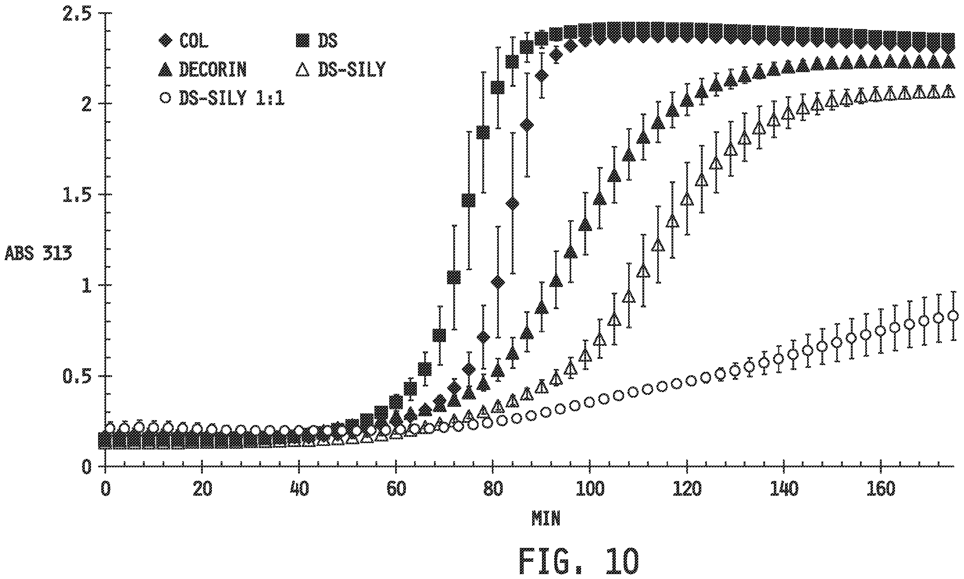

FIGURE 10. Turbidity measurement. Gel solutions were prepared as described in EXAMPLE 15 (collagen 4mg/mL and 10:1 collagen to treatment, unless otherwise indicated) and 50µL/well were added at 4°C to a 384-well plate. The plate was kept at 4°C for 4 hours before initiating fibril formation. A SpectraMax M5 at 37°C was used to measure absorbance at 313nm at 30s intervals for 6 hours. Col, no treatment, i.e., collagen alone; DS, collagen + dermatan sulfate; decorin, collagen + decorin; DS-SILY, collagen + dermatan sulfate-SILY conjugate.

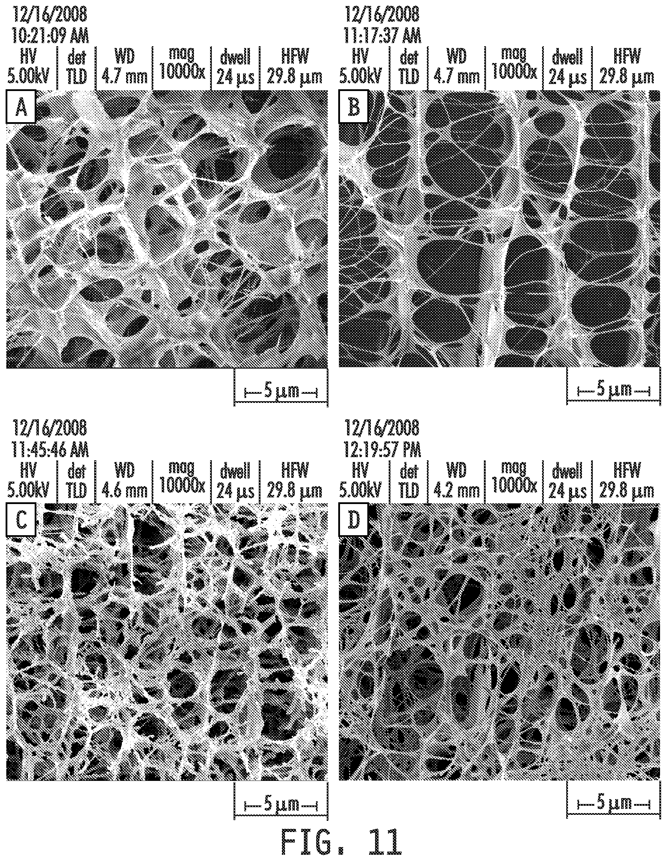

FIGURE 11. Cryo-Scanning Electron Microscopy images of gel structure at a magnification of 5000. Gels for cryo-SEM were formed, as described in EXAMPLE 18 (1 mg/mL collagen (Type III), 1:1 collagen:treatment), directly on the SEM stage. Regions with similar orientation were imaged for comparison across treatments. Panel a, Collagen, no treatment, i.e., collagen alone; Panel b, collagen + dermatan sulfate; Panel c, collagen + dermatan sulfate-KELN conjugate; Panel d, collagen + dermatan sulfate-GSIT conjugate.

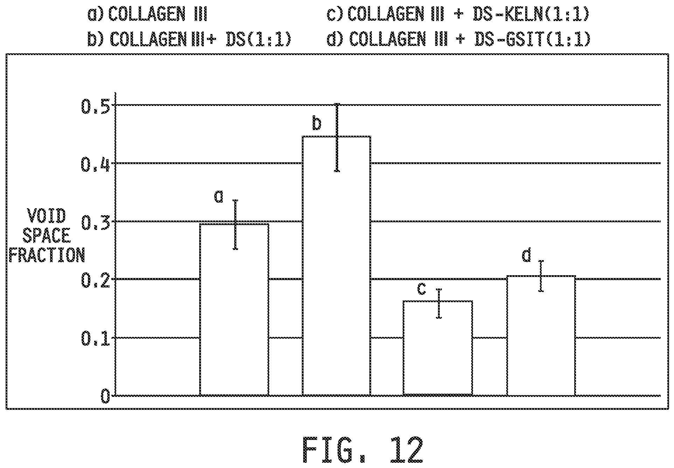

FIGURE 12. The average void space fraction measured from the Cryo-SEM images shown in FIGURE 11. a) Collagen, no treatment, i.e., collagen alone; b) collagen + dermatan sulfate; c) collagen + dermatan sulfate-KELN conjugate; d) collagen + dermatan sulfate-GSIT conjugate. All differences are significant with p=0.05.

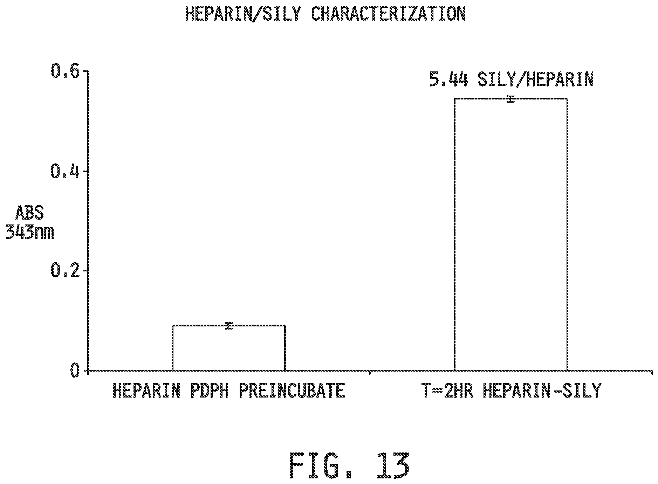

FIGURE 13. Measurement of absorbance at 343nm before treatment of oxidized heparin conjugated to PDPH, and after treatment with SILY, which releases 2-pyridylthiol from the conjugate and allows determination of the ratio of SILY peptide conjugated to oxidized heparin. The measured ΔA, corresponds to 5.44 SILY molecules/oxidized heparin.

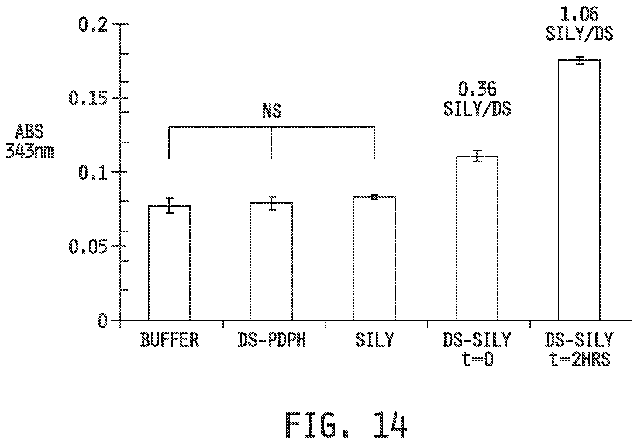

FIGURE 14. DS-SILY Conjugation Characterization. After 2 hours, a final ΔA₃₄₃ₙₘ corresponded to 1.06 SILY molecules added to each DS molecule. Note, t=0 is an approximate zero time point due to the slight delay between addition of SILY to the DS-PDPH and measurement of the solution at 343 nm.

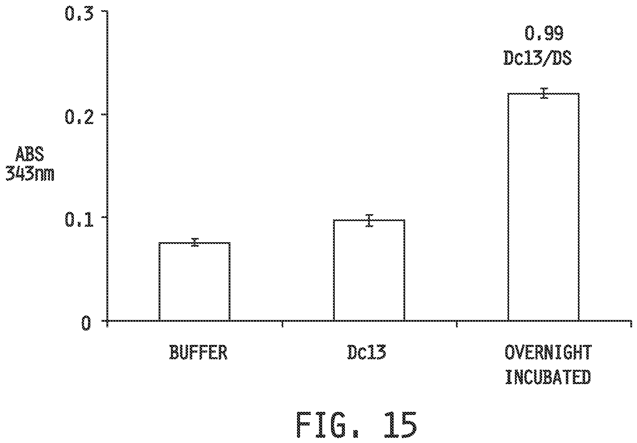

FIGURE 15. Conjugation of Dcl3 to DS. Production of pyridine-2-thione measured by an increase in absorbance at 343nm indicates 0.99 Dc13 peptides per DS polymer chain.

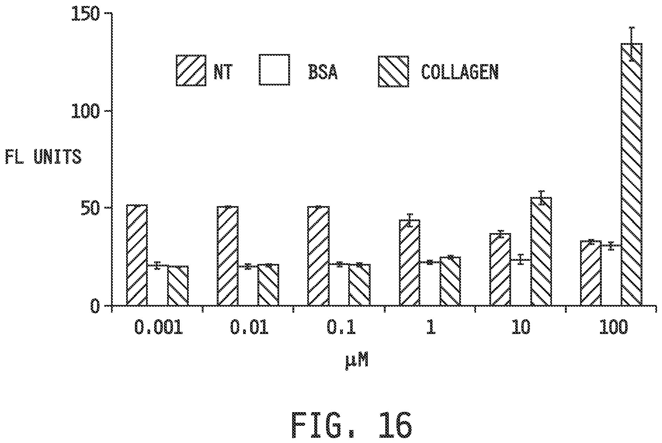

FIGURE 16. Microplate Fluorescence Binding of DS-ZDc13 to Collagen. DS-ZDc13 bound specifically to the collagen surface in a dose-dependent manner.

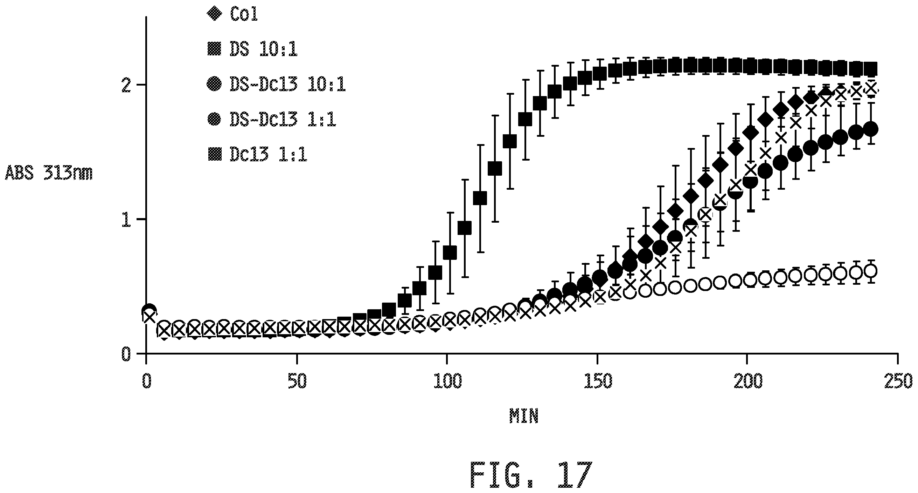

FIGURE 17. Collagen Fibrillogenesis by Turbidity Measurements. DS-Dc13 delays fibrillogenesis and decreases overall absorbance in a dose-dependent manner. Free Dc13 peptide, in contrast, appears to have little effect on fibrillogenesis compared to collagen alone at the high 1:1 collagen:additive molar ratio.

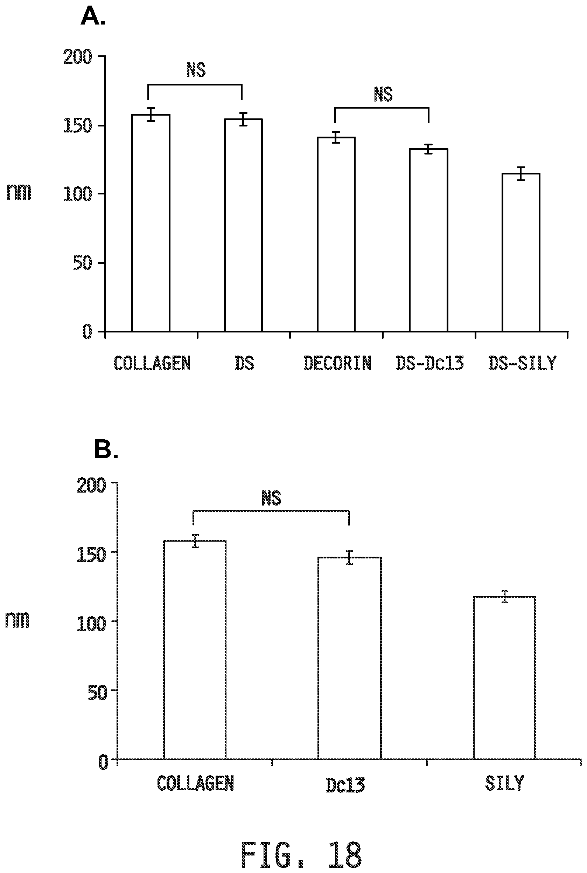

FIGURE 18. Average Fibril Diameter from Cryo-SEM. A. Decorin and synthetic peptidoglycans significantly decrease fibril diameter over collagen or collagen + DS. B. Compared to collagen alone, free peptide Dc13 does not affect fibril diameter while SILY results in a decrease in fibril diameter.

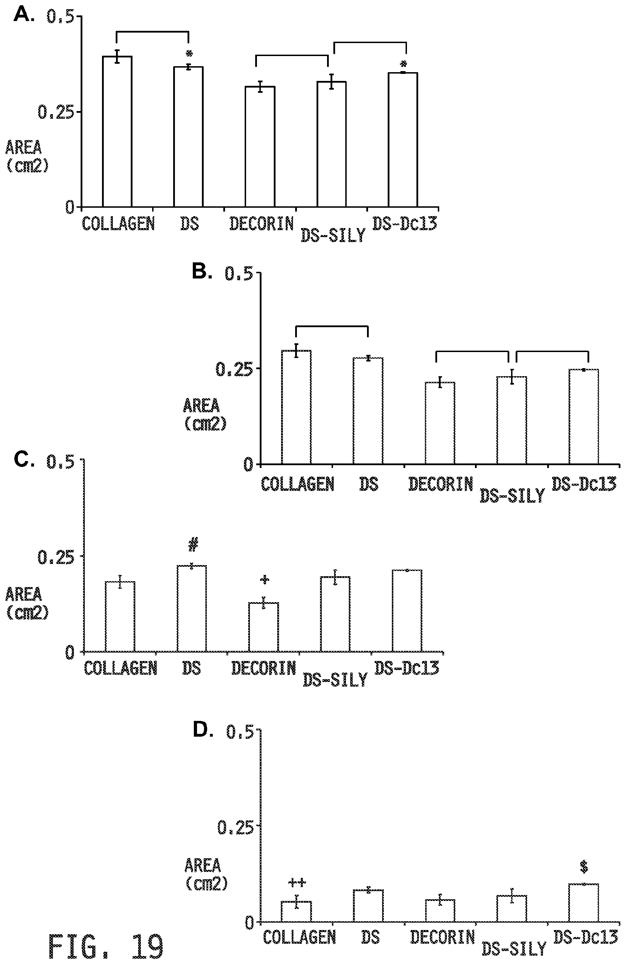

FIGURE 19. Gel Compaction. A. and B. Days 3 and 5 respectively: Decorin and peptidoglycans are significant relative to collagen and DS, * indicates DS-Dc13 and DS are not significant at day 3. Bars indicate no significance. C. Day 7: + Decorin is significant against all samples, # DS is significant compared to collagen. D. Day 10: ++ collagen and DS are significant, ‡ DS-Dc13 is significant compared to decorin and collagen.

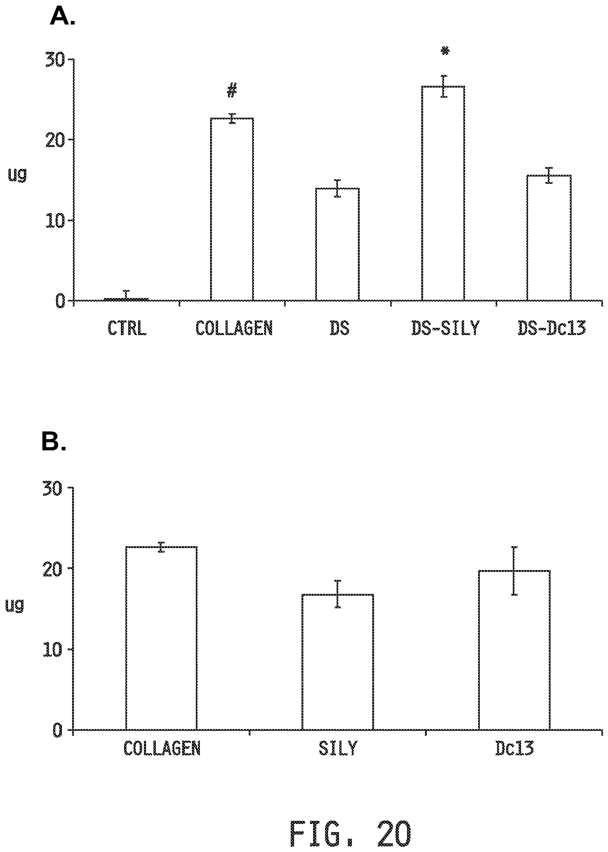

FIGURE 20. Elastin Estimate by Fastin Assay. A. DS-SILY significantly increased elastin production over all samples. DS and DS-Dc13 significantly decreased elastin production over collagen. Control samples of collagen gels with no cells showed no elastin production. B. Free peptides resulted in a slight decrease in elastin production compared to collagen, but no points were significant.

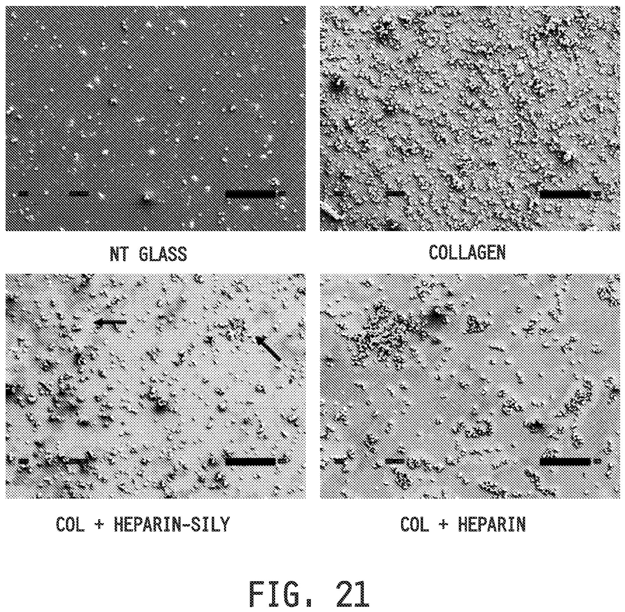

FIGURE 21. SEM Images of Platelet-Rich Plasma Incubated Slides. Arrows in Heparin-SILY treatment indicate fibril-like structures unique to this treatment. Scale bar = 100 µm.

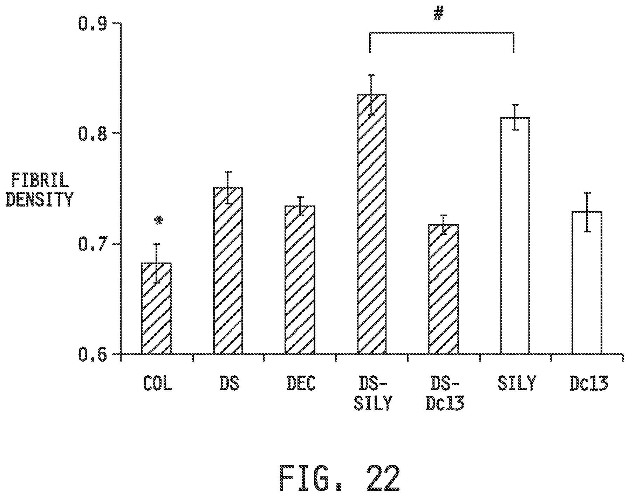

FIGURE 22. Fibril Density from Cryo-SEM. Fibril density, defined as the ratio of fibril containing area to void space. DS-SILY and free SILY peptide had significantly greater fibril density, while collagen had significantly lower fibril density. DS-Dc13 was not significant compared to collagen.

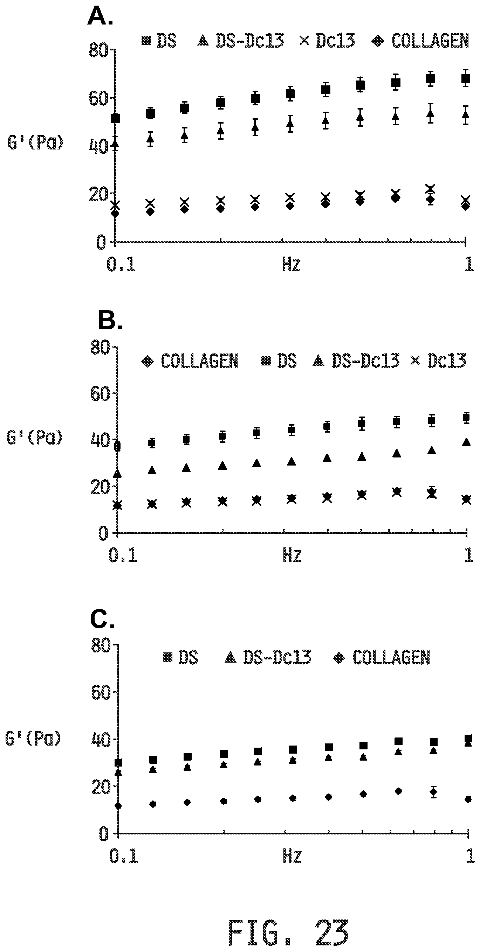

FIGURE 23. Storage Modulus (G') of Collagen Gels. Rheological mechanical testing of collagen gels formed with each additive at A. 5:1 B. 10:1 and C. 30:1 molar ratio of collagen:additive. Frequency sweeps from 0.1 Hz to 1.0 Hz with a controlled stress of 1.0 Pa were performed. G'avg ± S.E. are presented.

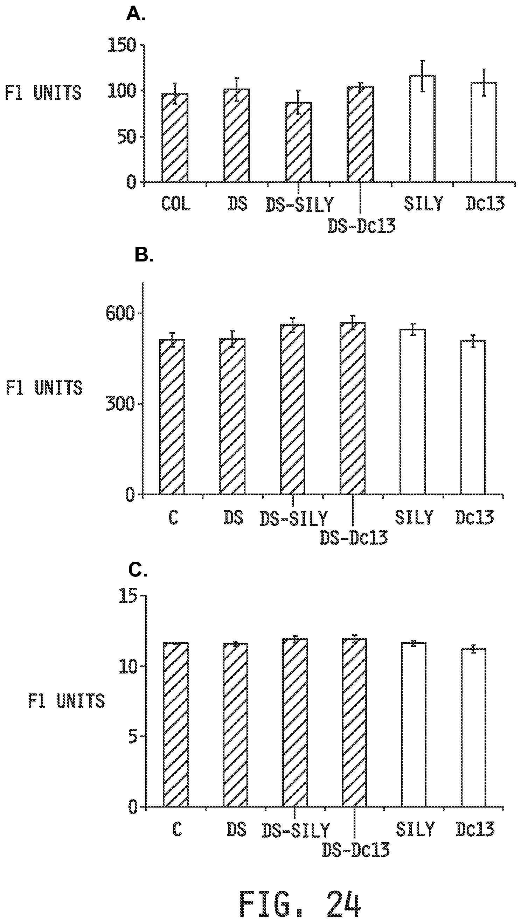

FIGURE 24. Cell Proliferation and Cytotoxicity Assays. No significant differences were found between all additives in A. CyQuant B. Live and C. Dead assays.

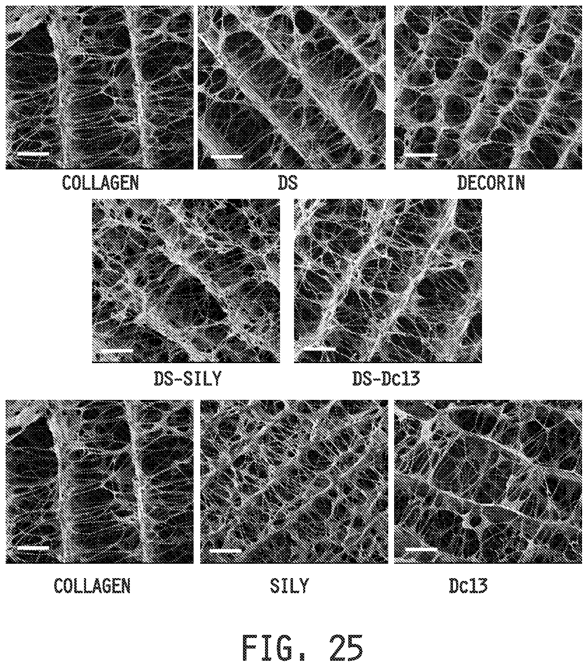

FIGURE 25. Cryo-SEM Images for Fibril Density. Collagen gels formed in the presence of each additive at a 10:1 molar ratio of collagen:additive. A. DS, Decorin, or peptidoglycans. B. Free Peptides. Images are taken at 10,000x, Scale bar = 5 µm.

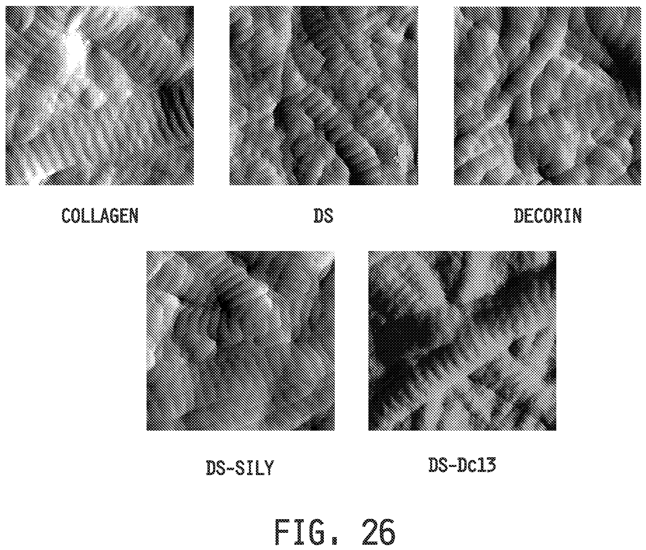

FIGURE 26. AFM Images of Collagen Gels. Collagen gels were formed in the presence of each additive at a 10:1 molar ratio of collagen:additive. D-banding is observed for all additives. Images are 1 µm².

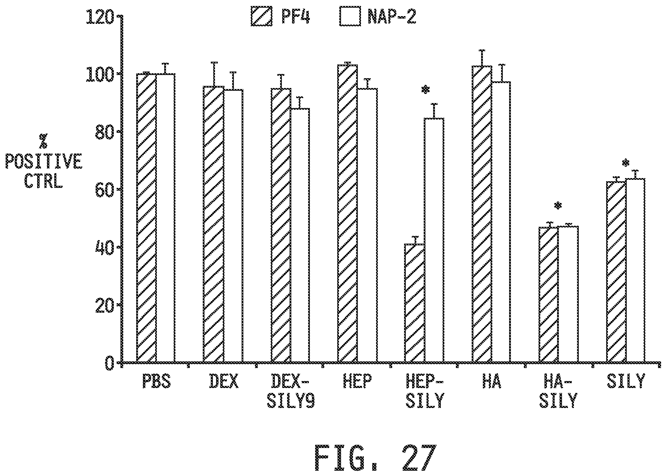

FIGURE 27. Inhibition of Platelet Activation. Measured by determining the release of activation factors Platelet Factor 4 (PF-4) and β-thromboglobulin (Nap-2). Collagen immobilized on the surface of a 96-well plate was pre-incubated with each treatment and subsequently incubated with platelet rich plasma (PRP). Values are reported as a percentage of activation factor released by the treatment compared to the amount of activation factor released by the control treatment (phosphate buffered saline, PBS). The * indicates that the difference is significant vs. collagen surface with no treatment (phosphate buffered saline, PBS). Dex, dextran; Dex-SILY9, dextran-(SILY)₉ conjugate; Hep, heparin; Hep-SILY, heparin-SILY conjugate; HA, hyaluronan; HA-SILY, hyaluronan-SILY conjugate; SILY, SILY peptide. Due to solubility limits, Hep, Hep-SILY, HA, and HA-SILY were incubated at 25µM. All other treatments were at 50µM (after the treatment was removed, the plates were washed with PBS < 1 min, before addition of PRP). Hep and HA (hyaluronic acid) conjugates contained approximately 4 peptides per polysaccharide.

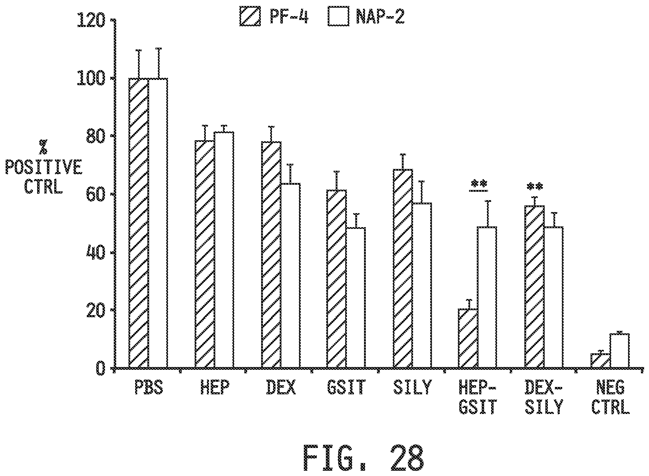

FIGURE 28. Inhibition of Platelet Activation. Measured by determining the release of activation factors Platelet Factor 4 (PF-4) and β-thromboglobulin (Nap-2). Collagen immobilized on the surface of a 96-well plate was pre-incubated with each treatment and subsequently incubated with platelet rich plasma (PRP). Values are reported as a percentage of activation factor released by the treatment compared to the amount of activation factor released by the control treatment (phosphate buffered saline, PBS). Dex, dextran; Dex-SILY6, dextran-(SILY)₆ conjugate; Hep, heparin; Hep-GSIT, heparin-GSIT conjugate; GSIT, GSIT peptide; SILY, SILY peptide. The values measured for all treatments are significant vs. PBS. Dex, SILY, and Dex-SILY6 are at 25µM, all other treatments are at 50µM. The ** indicates that the value for the Hep-GSIT treatment was significant vs. the values for the Hep treatment, similarly the value for the Dex-SILY6 treatment was significant vs. the value for the Dex treatment for PF4. (After the treatment was removed the plates were rinsed for 20 min). Hep conjugates contained approximately 4 peptides per polysaccharide.

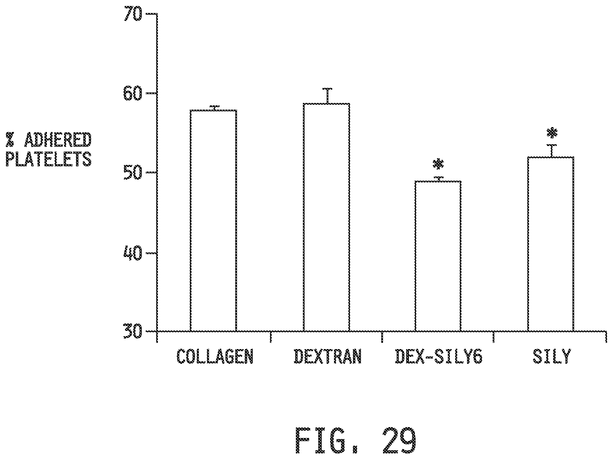

FIGURE 29. Inhibition of Platelet Binding to Collagen by Colorimetric Assay. Collagen immobilized on the surface of a 96-well plate was pre-incubated with each treatment and subsequently incubated with platelet rich plasma (PRP). Microplate assay prepared as described was pre-incubated with treatments Collagen, PBS only; Dextran; Dex-SILY6, dextran-(SILY)₆; SILY, SILY peptide. * Significant vs. collagen (no treatment).

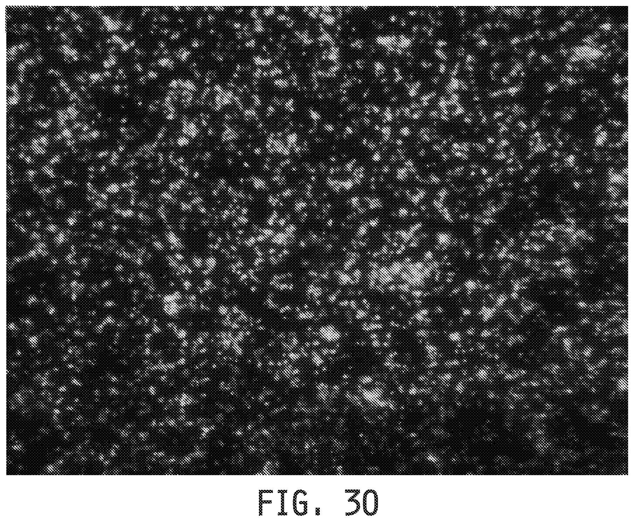

FIGURE 30. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescent microscope using a DAPI filter. No treatment, i.e. collagen treated with PBS.

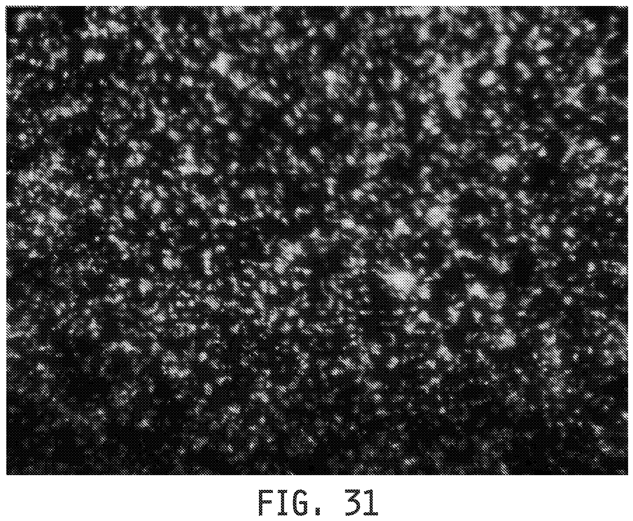

FIGURE 31. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescence microscope using a DAPI filter. Treatment: dextran.

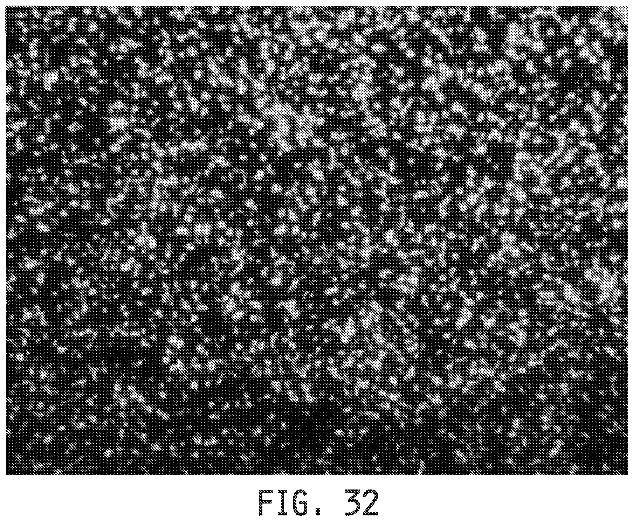

FIGURE 32. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescence microscope using a DAPI filter. Treatment: dextran-SILY9 conjugate.

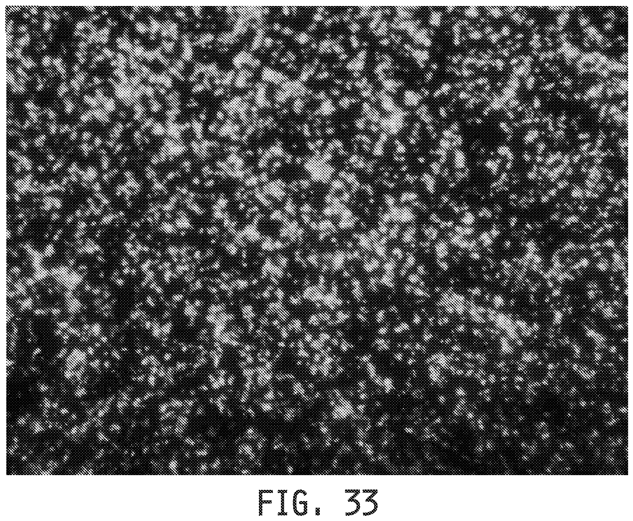

FIGURE 33. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescence microscope using a DAPI filter. Treatment: hyaluronan.

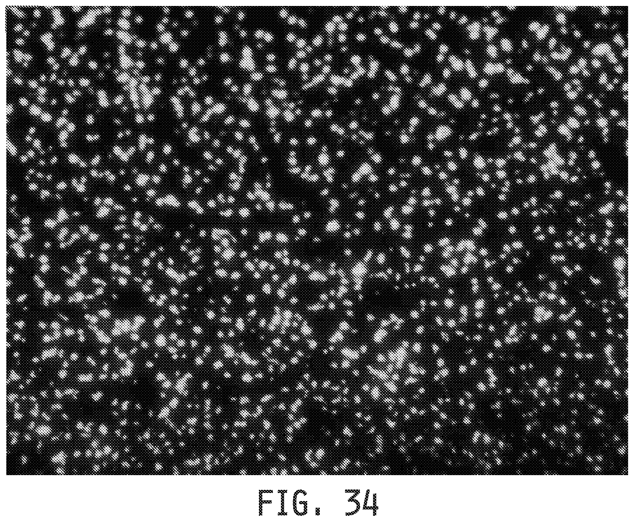

FIGURE 34. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescence microscope using a DAPI filter. Treatment: hyaluronan-SILY conjugate.

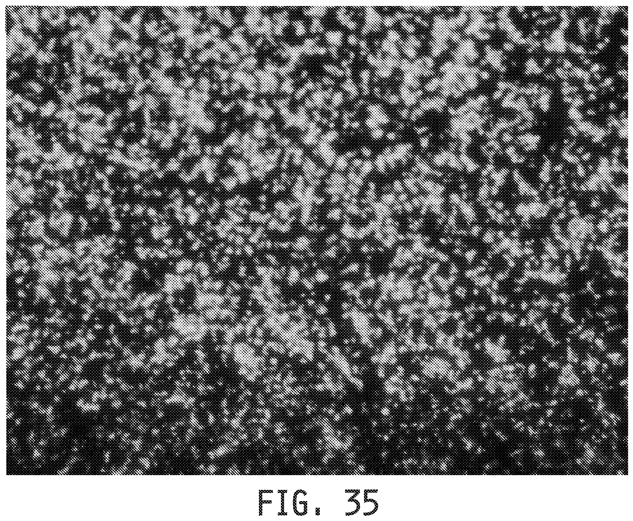

FIGURE 35. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescence microscope using a DAPI filter. Treatment: heparin.

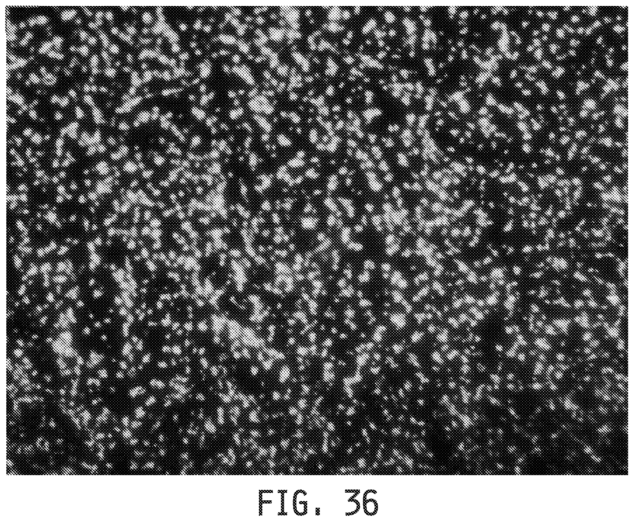

FIGURE 36. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescence microscope using a DAPI filter. Treatment: heparin-SILY conjugate.

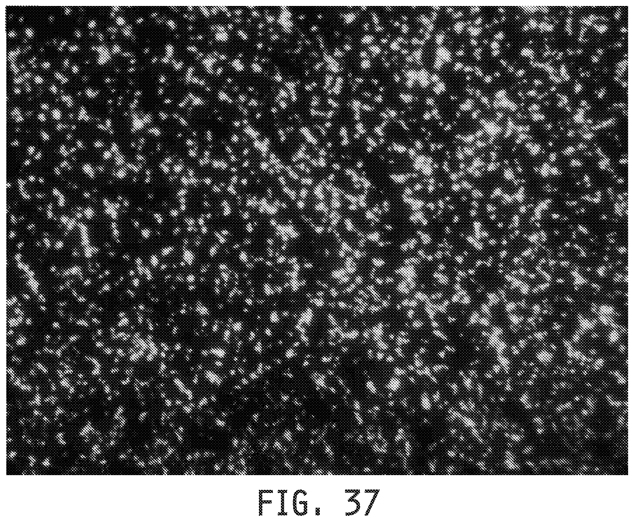

FIGURE 37. Fluorescence image of adhered platelets. Adhered platelets were fixed with 4% paraformaldehyde, permeabilized with 0.1% Triton X-100, and platelet actin was labeled with phalloidin-AlexaFluor 488. The adhered platelets were imaged using an upright fluorescence microscope using a DAPI filter. Treatment: SILY peptide.

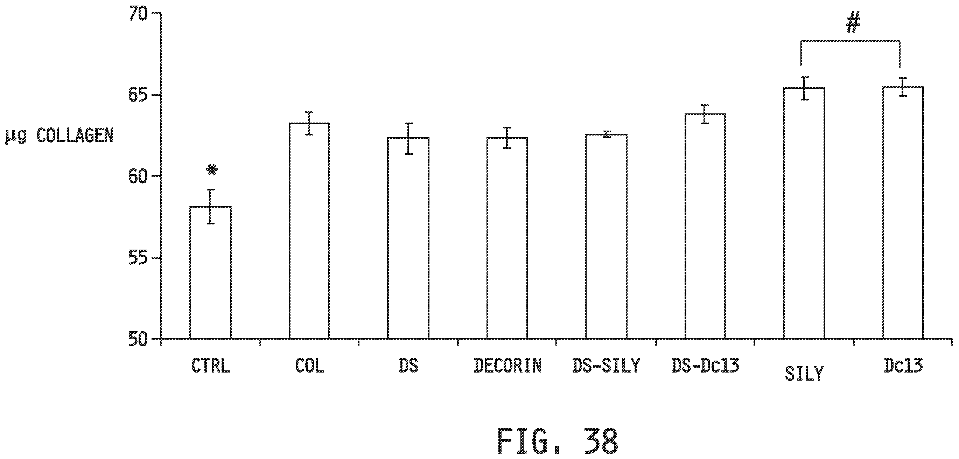

FIGURE 38. Collagen Degradation Determined by Hydroxyproline. Treatments: Ctrl, no cells added; Col, collagen without added treatment; DS, dermatan sulfate; Decorin; DS-SILY, dermatan sulfate-SILY conjugate; DS-Dc13, dermatan sulfate-Dc13 conjugate; SILY, SILY peptide; Dcl3, Dc13 peptide.

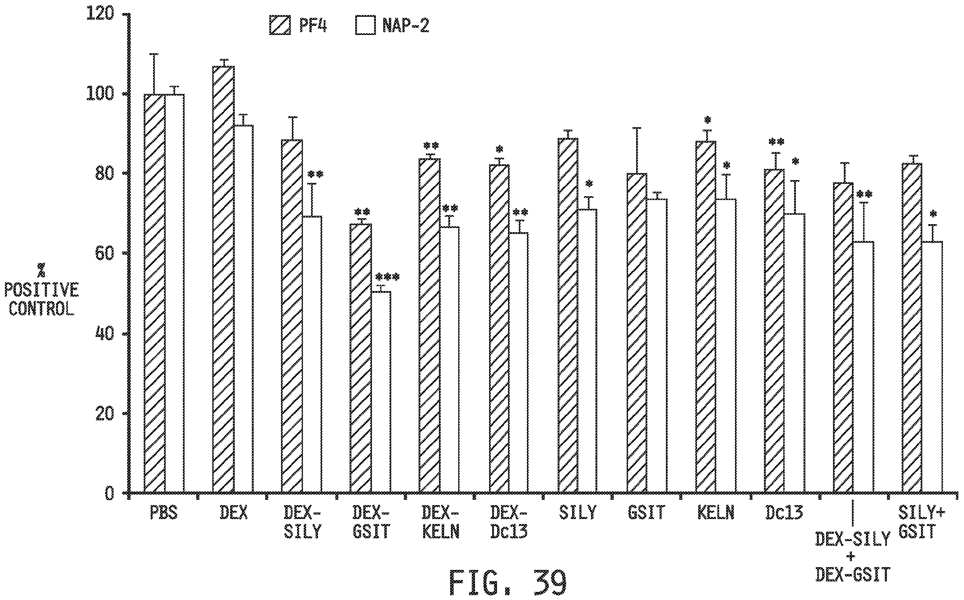

FIGURE 39. Inhibition of Platelet Activation. Measured by determining the release of activation factors Platelet Factor 4 (PF-4) and β-thromboglobulin (Nap-2). Type I and III collagen gels on the surface of a 96-well plate were pre-incubated with each treatment and subsequently incubated with PRP. Platelet activation was measured by the release of activation factors PF-4 and Nap-2. Treatments: PBS, buffer alone; Dex, dextran; Dex-SILY, dextran-SILY conjugate; Dex-GSIT, dextran-GSIT conjugate; Dex-KELN, dextran-KELN conjugate; Dex-Dcl3, dextran-Dcl3 conjugate; SILY, SILY peptide; GSIT, GSIT peptide; KELN, KELN peptide; Dc13, Dc13 peptide; Dex-SILY+Dex-GSIT; combination of dextran-SILY conjugate and dextran-GSIT conjugate; SILY+GSIT; combination of SILY peptide and GSIT peptide. * Indicates the results are significant vs. collagen surface with no treatment (PBS). ** Indicates the results are also significant vs. collagen surface with Dex. *** Indicates the results are also significant vs. collagen surface with corresponding peptide control. All peptidoglycans caused significant decrease in NAP-2 release compared to no treatment (PBS) or dextran treatment, while Dex-GSIT additionally decreased release over its peptide control (GSIT). Dex-GSIT and Dex-KELN significantly decreased PF-4 release relative to no treatment (PBS) and dextran treatment, while Dex-Dc13 significantly decreased PF-4 release over no treatment (PBS).

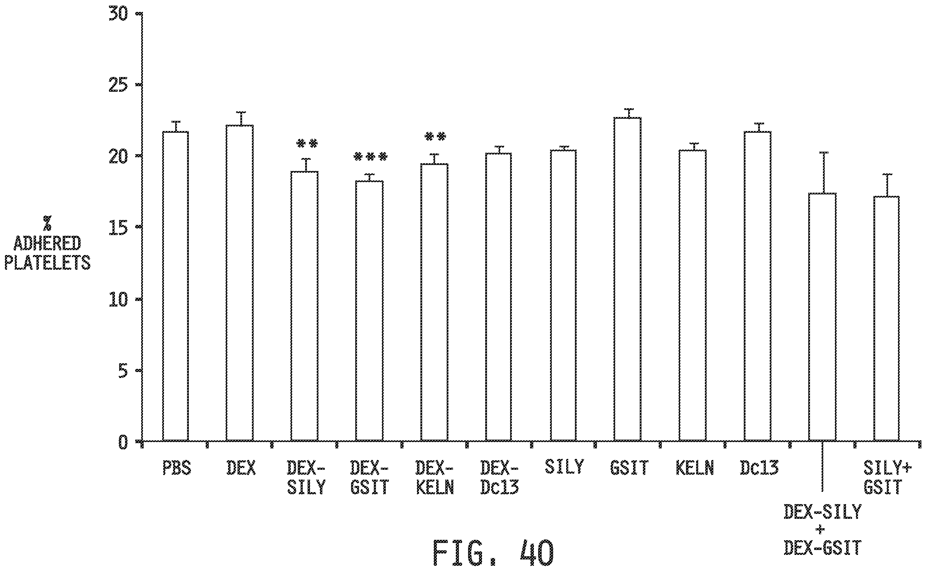

FIGURE 40. Inhibition of Platelet Binding to Collagen (Adhesion) by Colorimetric Assay. Treatments: PBS, buffer alone; Dex, dextran; Dex-SILY, dextran-SILY conjugate; Dex-GSIT, dextran-GSIT conjugate; Dex-KELN, dextran-KELN conjugate; Dex-Dc13, dextran-Dc13 conjugate; SILY, SILY peptide; GSIT, GSIT peptide; KELN, KELN peptide; Dc13, Dc13 peptide; Dex-SILY+Dex-GSIT; combination of dextran-SILY conjugate and dextran-GSIT conjugate; SILY+GSIT; combination of SILY peptide and GSIT peptide. * Significant vs. Collagen surface with no treatment (PBS). ** Also significant vs. collagen surface with Dex. *** Also significant vs. collagen surface with corresponding peptide control. Dex-SILY and Dex-KELN had significantly decreased platelet adherence as compared to no treatment (PBS) or Dextran treatment, while Dex-GSIT additionally decreased platelet adherence over its peptide control treatment (GSIT).

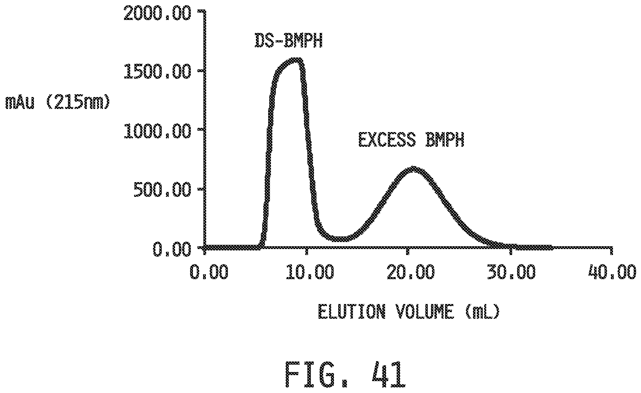

FIGURE 41 shows the purification of DS-BMPH. The number of BMPH crosslinkers attached to DS is determined by calculating the excess BMPH which is then subtracted from the known amount added, which yields the amount reacted with oxidized DS. Excellent separation of the two molecular species is achieved under the purification procedures, which is shown by the wide separation of peaks.

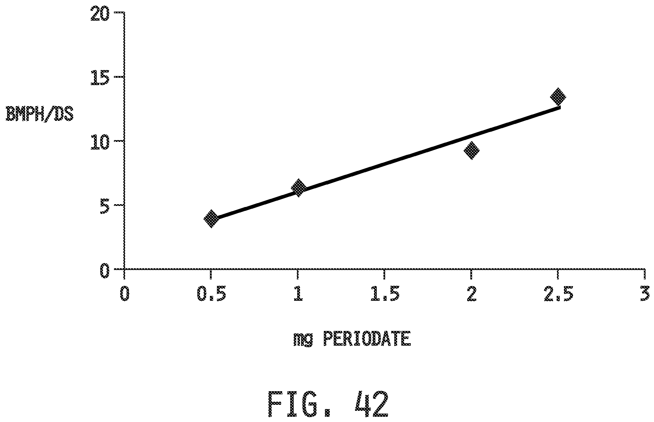

FIGURE 42 shows periodate oxidation. By increasing the amount of sodium meta-periodate during oxidation of DS, the number of BMPH crosslinkers per DS chain increases linearly.

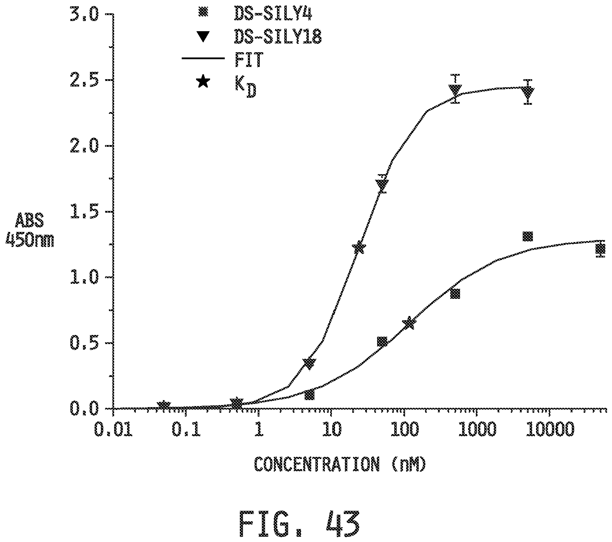

FIGURE 43 shows peptidoglycan binding affinity. Biotin labeled peptidoglycans DS-SILY₄ and DS-SILY₁₈ were synthesized and incubated on a fibrillar collagen surface. After washing, the bound peptidoglycan was detected and saturation binding curves were fitted to calculate the binding affinities. DS-SILY₄ and DS-SILY₁₈ binding to collagen with K_{D}=118nM and 24nM respectively, demonstrating that increasing the number of attached peptides increases the affinity of the peptidoglycan to collagen. In addition a greater number of peptides increases the amount of peptidoglycan that binds to the surface, which is noted by the increase in absorbance. Note DS-SILY₁₈ does not contain more biotin labeled peptides than DS-SILY₄.

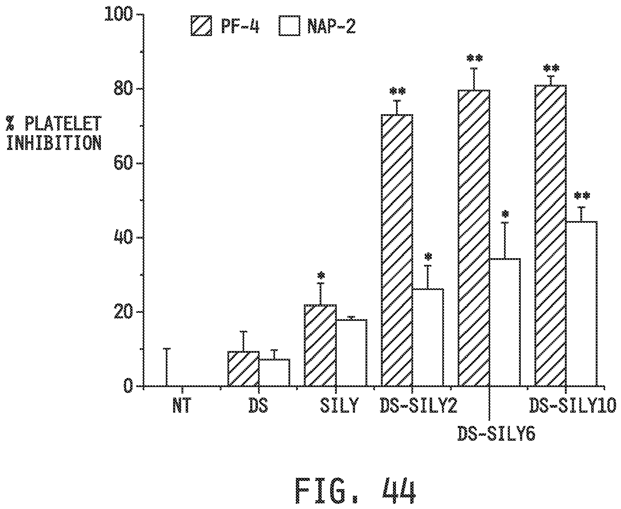

FIGURE 44 shows the percent decrease in release of activation factors PF4 and NAP2 as compared to untreated collagen surfaces (NT). DS, SILY, or DS-SILY was incubated for 15 min at a concentration of 50 µM and then rinsed from the collagen surface for 24 hours. * indicates significance to NT, ** indicates significance to NT, and DS, and SILY α = 0.05.

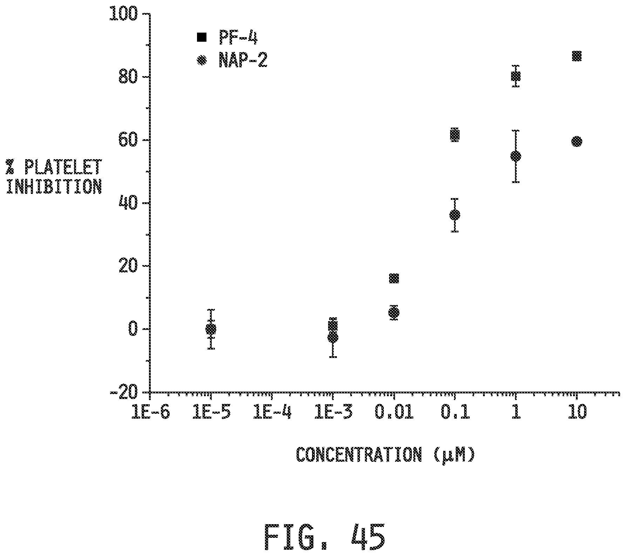

FIGURE 45 shows the inhibition of platelet activation. Fibrillar collagen surfaces were incubated with varying concentrations of peptidoglycan DS-SILY₁₈. Unbound peptidoglycan was rinsed from the surface over 24 hours. Human platelets were then incubated on the surface and activation was measured by release of PF-4 and Nap-2 following FDA guidelines. Maximal inhibition of platelet activation was achieved at 10 µM concentrations.

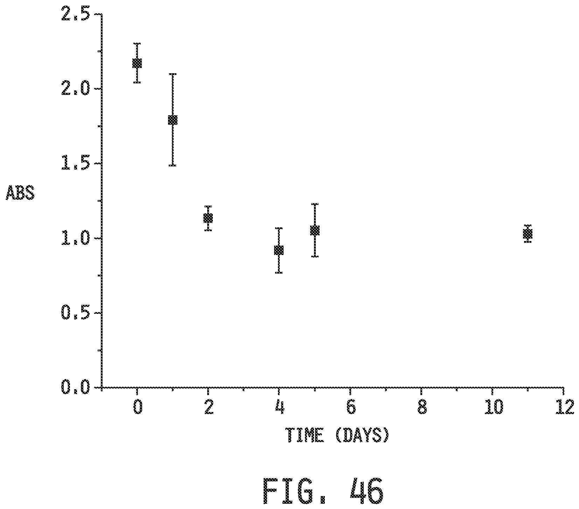

FIGURE 46 shows the diffusion of DS-SILY₁₈ from a fibrillar collagen surface. Labeled DS-SILY₁₈ was bound on a collagen surface as described and incubated at 37° C with extensive rinsing for up to 11 days. Detection of the peptidoglycan at various time points showed that it diffuses from the surface over time but even after 1 week, the equivalent of approximately 10 nM remained bound.

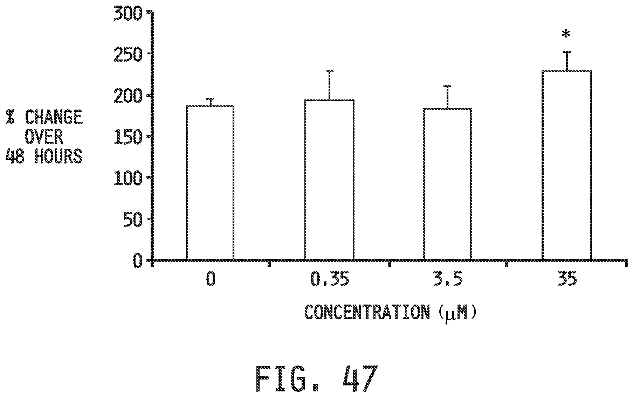

FIGURE 47 shows endothelial cell proliferation. Proliferation was measured in the presence of varying concentrations of peptidoglycan to determine whether the peptidoglycan had an adverse effect on endothelial regrowth. At the highest concentration tested, a significant increase in cell proliferation was observed. * indicates significance α=0.05, n = 6, presented as average + std. dev.

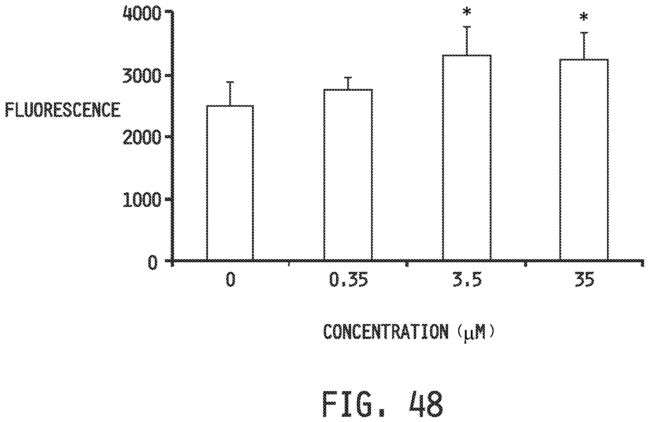

FIGURE 48 shows endothelial migration on treated collagen surfaces. To more closely mimic the environment of a denuded vessel with exposed collagen, endothelial growth onto collagen with varying concentrations of bound peptidoglycan was tested. At higher peptidoglycan concentrations there was a significant increase in endothelial cell migration. * indicates significance α=0.05, n = 6, presented as avg. + std dev.

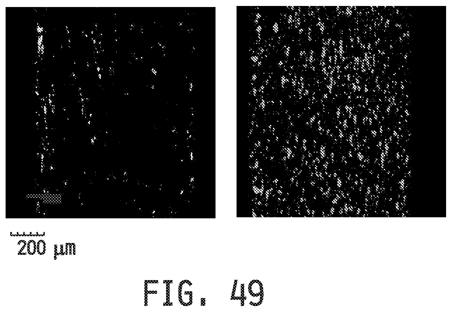

FIGURE 49 shows platelet binding to collagen under flow. Human platelet-rich plasma was tested under flow for platelet binding on fibrillar collagen surfaces. Treatment conditions DS-SILY treated (Panel A) or untreated (Panel B) collagen surfaces show significantly fewer bound platelets on the peptidoglycan treated surface.

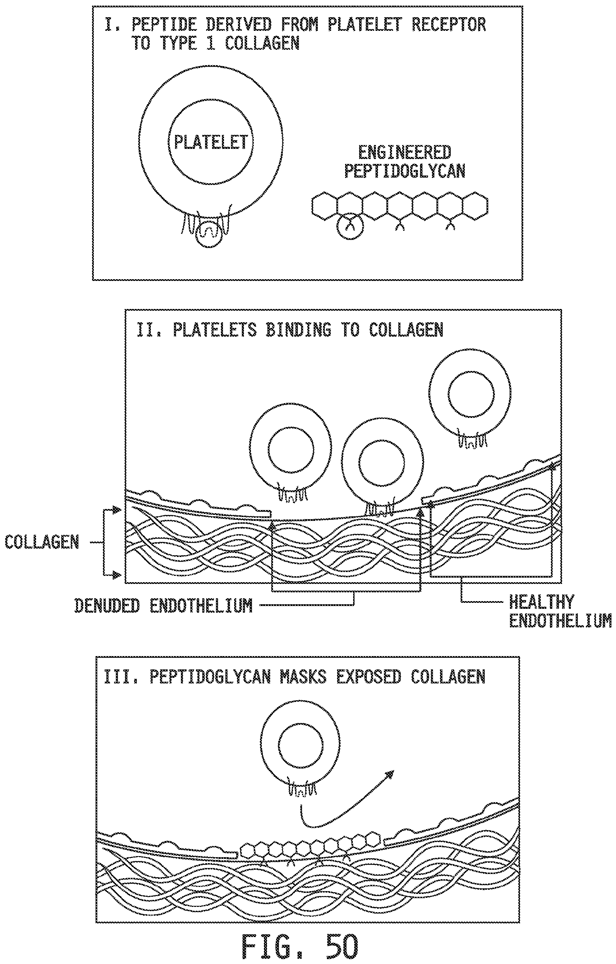

FIGURE 50 shows a schematic representation of peptidoglycan inhibition of platelet binding and activation on collagen of denuded endothelium.

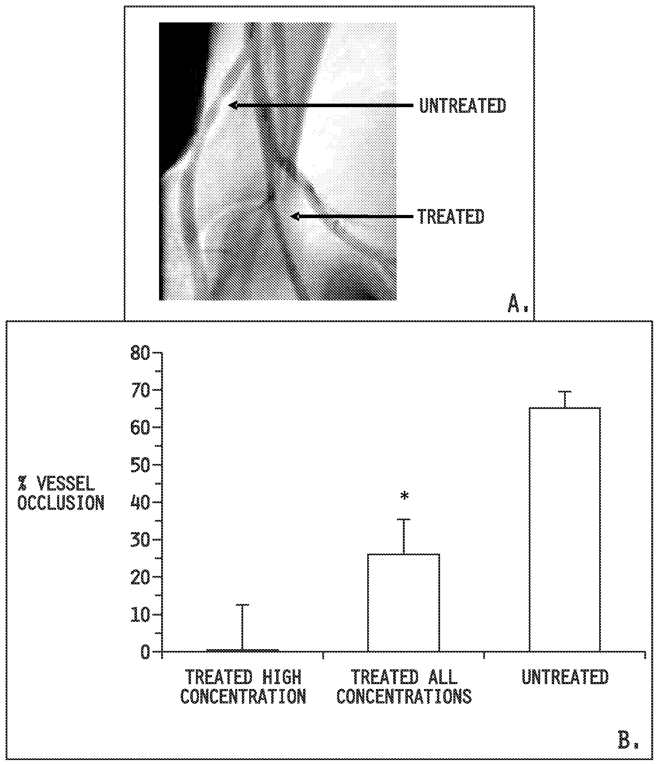

FIGURE 51 shows the quantification of inhibited platelet binding by vasospasm. Panel A shows a representative angiography profile of treated and untreated balloon injured vessels. Vasospasm is apparent in the untreated vessel while the peptidoglycan treatment does not exhibit vasospasm. Panel B shows vasospasm quantified by measuring the % vessel occlusion using angiography data. A total of 12 balloon injuries, 7 untreated and 5 treated, were analyzed. * indicates significance compared to untreated p=0.005.

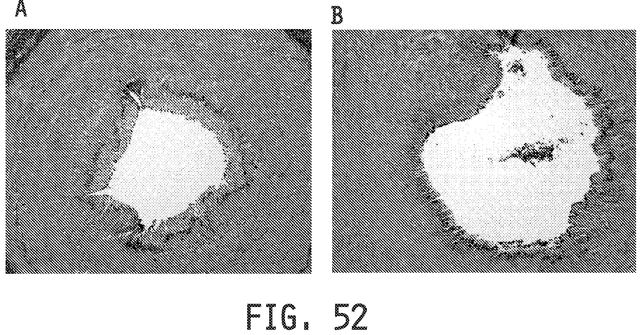

FIGURE 52 shows histological evaluation of balloon injured vessels using Verhoff-Van Gieson staining. Intimal hyperplasia is apparent in the sham control (panel A) as noted by growth from the internal elastic lamina. In peptidoglucan treated vessels, intimal hyperplasia is absent (panel B).

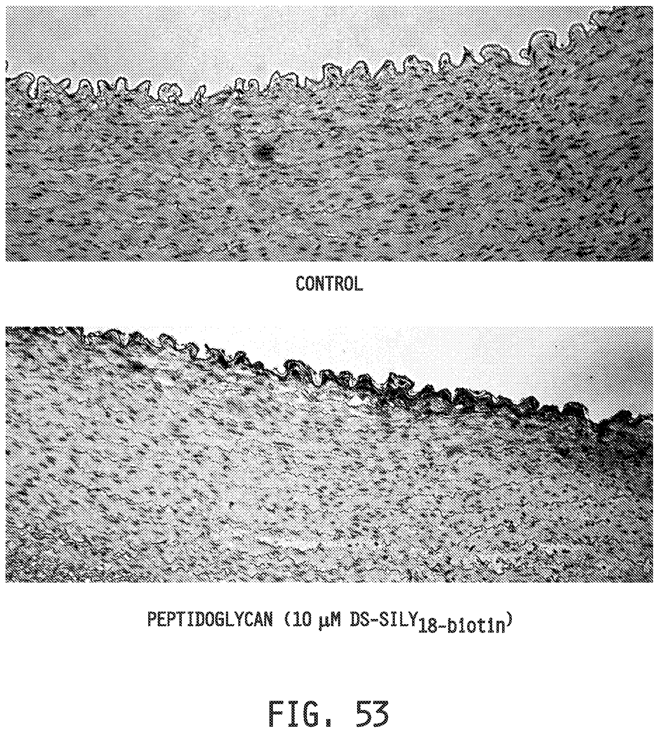

FIGURE 53 shows denuded arteries incubated with Ix PBS (Panel A: Control) or labeled peptidoglycan (Panel B: Peptidoglycan (10 µM DS-SILY_{18-biotin})).

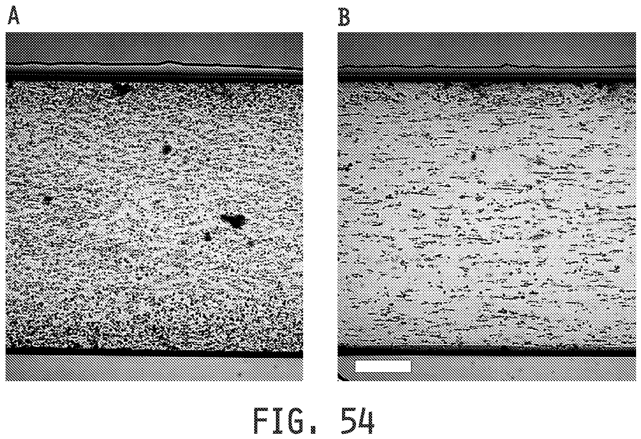

FIGURE 54 shows inhibition by DS-SILY18 of whole blood binding to collagen under flow.

### DETAILED DESCRIPTION OF THE ILLUSTRATIVE EMBODIMENTS

As used in accordance with this invention, a "collagen-binding synthetic peptidoglycan" means a collagen-binding conjugate of a glycan with a synthetic peptide. The "collagen-binding synthetic peptidoglycans" can have amino acid homology with a portion of a protein or a proteoglycan not normally involved in collagen fibrillogenes or can have amino acid homology to a portion of a protein or to a proteoglycan normally involved in collagen fibrillogenesis.

These collagen-binding synthetic proteoglycans can be used in vascular intervention procedures including, for example, to prevent any one or a combination of platelet binding to exposed collagen of the denuded endothelium, platelet activation, thrombosis, inflammation resulting from denuding the endothelium, intimal hyperplasia, and vasospasm. The collagen-binding synthetic peptidoglycans described herein can also stimulate endothelial cell proliferation and can bind to collagen in a denuded vessel.

In various embodiments described herein, the collagen-binding synthetic peptidoglycans described comprise synthetic peptides of about 5 to about 40 amino acids. In some embodiments, these peptides have homology to the amino acid sequence of a small leucine-rich proteoglycan, a platelet receptor sequence, or a protein that regulates collagen fibrillogenesis.
The synthetic peptide is an amino acid sequence of RRANAALKAGELYKSILY, and an amino acid sequence with 80%, 85%, 90%, 95%, or 98% homology with to the amino acid sequence. In a disclosure not within the scope of the invention, the synthetic peptide can also be any peptide of 5 to 40 amino acids selected from peptides that have collagen-binding activity and that are 80%, 85%, 90%, 95%, 98%, or 100% homologous with the collagen-binding domain(s) of the von Willebrand factor or a platelet collagen receptor as described in Chiang, et al.. J Biol. Chem. 277: 34896-34901 (2002), Huizinga, et al., Structure 5: 1147-1156 (1997), Romijn, et al., J Biol. Chem. 278: 15035-15039 (2003), and Chiang, et al., Cardio. & Haemato. Disorders-Drug Targets 7: 71-75 (2007).

The glycan (e.g. glycosaminoglycan, abbreviated GAG, or polysaccharide) attached to the synthetic peptide can be selected from the group consisting alginate, agarose, dextran, chondroitin, dermatan, dermatan sulfate, heparan, heparin, keratin, and hyaluronan. In one embodiment, the glycan is selected from the group consisting of dermatan sulfate, dextran, and heparin. In another illustrative embodiment the glycan is dermatan sulfate. The collagen- binding synthetic proteoglycan in any of these embodiments can be used to inhibit platelet binding to exposed collagen of the denuded endothelium, platelet activation, thrombosis, inflammation resulting from denuding the endothelium, intimal hyperplasia, and vasospasm during a vascular intervention procedure. The collagen-binding synthetic peptidoglycans described herein can also stimulate endothelial cell proliferation and can bind to collagen in a denuded vessel.

In one illustrative aspect, the collagen-binding synthetic peptidoglycan may be sterilized. As used herein "sterilization" or "sterilize" or "sterilized" means disinfecting the collagen-binding synthetic peptidoglycans by removing unwanted contaminants including endotoxins and infectious agents.

In various illustrative embodiments, the collagen-binding synthetic peptidoglycan can be disinfected and/or sterilized using conventional sterilization techniques including propylene oxide or ethylene oxide treatment, gas plasma sterilization, gamma radiation, electron beam, and/or sterilization with a peracid, such as peracetic acid.
Sterilization techniques which do not adversely affect the structure and biotropic properties of the collagen-binding synthetic peptidoglycan can be used. Illustrative sterilization techniques are exposing the collagen-binding synthetic peptidoglycan to peracetic acid, 1-4 Mrads gamma irradiation (or 1-2.5 Mrads of gamma irradiation), ethylene oxide treatment, sterile filtration, or gas plasma sterilization. In one embodiment, the collagen-binding synthetic peptidoglycan can be subjected to one or more sterilization processes. Another illustrative embodiment is subjecting the collagen-binding synthetic proteoglycan to sterile filtration. The collagen- binding synthetic peptidoglycan may be wrapped in any type of container including a plastic wrap or a foil wrap, and may be further sterilized.

In various embodiments described herein, the collagen-binding synthetic peptidoglycans can be combined with minerals, amino acids, sugars, peptides, proteins, vitamins (such as ascorbic acid), or laminin, collagen, fibronectin, hyaluronic acid, fibrin, elastin, or aggrecan, or growth factors such as epidermal growth factor, platelet-derived growth factor, transforming growth factor beta, or fibroblast growth factor, and glucocorticoids such as dexamethasone or viscoelastic altering agents, such as ionic and non-ionic water soluble polymers; acrylic acid polymers; hydrophilic polymers such as polyethylene oxides, polyoxyethylene-polyoxypropylene copolymers, and polyvinylalcohol; cellulosic polymers and cellulosic polymer derivatives such as hydroxypropyl cellulose, hydroxyethyl cellulose, hydroxypropyl methylcellulose, hydroxypropyl methylcellulose phthalate, methyl cellulose, carboxymethyl cellulose, and etherified cellulose; poly(lactic acid), poly(glycolic acid), copolymers of lactic and glycolic acids, or other polymeric agents both natural and synthetic.

In various embodiments described herein, a kit is provided comprising one or more collagen-binding synthetic peptidoglycans. The kit itself can be within a container of any type, and the kit can contain instructions for use of the components of the kit. In one embodiment, the kit comprises a vessel, vial, container, bag, or wrap, for example, containing a collagen-binding synthetic peptidoglycan. In another embodiment, the kit comprises a vessel or separate vessels (e.g., a vial, container, bag, or wrap), each containing one of the following
components: a buffer and one or more types of collagen-binding synthetic peptidoglycans. In any of these embodiments, the kits can further comprise a buffer, a sterilizing or disinfecting agent, non-collagenous proteins or polysaccharides, and/or instructional materials describing methods for using the kit reagents. In any of these embodiments, the kit can contain a component selected from the group consisting of a catheter, a stent, a balloon, and a combination thereof. The collagen-binding synthetic peptidoglycan can be lyophilized, for example, in a buffer or in water.

In any of the embodiments herein described, the collagen-binding synthetic peptidoglycan can be a compound of the following formula
(PₙL)ₓG wherein n is 1 to 7;
wherein x is 1 to 10;
wherein P is a synthetic peptide of about 5 to about 40 amino acids comprising a sequence of RRANAALKAGELYKSILY;
wherein L is a linker; and
wherein G is a glycan.

In the above described formula, n can be 1 to 5.

In various embodiments described herein, a collagen-binding synthetic peptidoglycan comprising a synthetic peptide of about 5 to about 40 amino acids with amino acid sequence homology to a collagen binding peptide (e.g. a portion of an amino acid sequence of a collagen binding protein or proteoglycan) conjugated to alginate, agarose, dextran, chondroitin, dermatan, dermatan sulfate, heparan, heparin, keratin, and hyaluronan. In one embodiment, the glycan is selected from the group consisting of dermatan sulfate, dextran, hyaluronan, and heparin. In another illustrative embodiment the glycan is dermatan sulfate. In
yet another embodiment, the glycan is dermatan sulfate with 18 peptides of the sequence RRANAALKAGELYKSILYGC linked to the glycan (i.e., DS-SILY₁₈). In yet another embodiment, the glycan is dermatan sulfate with 18 peptides comprising the sequence RRANAALKAGELYKSILY linked to the glycan. The collagen-binding synthetic proteoglycan in any of these embodiments can be used to inhibit platelet binding to exposed collagen of the denuded endothelium, inhibit binding of other cells in blood to exposed collagen of the denuded epithelium, inhibit platelet activation, inhibit thrombosis, inhibit inflammation resulting from denuding the endothelium, inhibit intimal hyperplasia, and/or inhibit vasospasm. The collagen-binding synthetic peptidoglycans described herein can also stimulate endothelial cell proliferation and can bind to collagen in a denuded vessel. In any of these embodiments, these aforementioned effects can occur during a vascular intervention procedure, such as a catheter-based procedure. In any of these embodiments, any of the above-described compounds can be used.

In another illustrative embodiment, any of the compounds described above as embodiments A, B, or C or alternative embodiments A or B can inhibit platelet binding to exposed collagen of the denuded endothelium, platelet activation, thrombosis, inflammation resulting from denuding the endothelium, intimal hyperplasia, and/or vasospasm, or can stimulate endothelial cell proliferation or can bind to collagen in a denuded vessel. In another illustrative embodiment, during a vascular intervention procedure, any of the compounds described above as embodiments A, B, or C or alternative embodiments A or B, can inhibit platelet binding to exposed collagen of the denuded endothelium, platelet activation, thrombosis, inflammation resulting from denuding the endothelium, intimal hyperplasia, and/or vasospasm, or can stimulate endothelial cell proliferation or can bind to collagen in a denuded vessel. In another illustrative embodiment, during a vascular intervention procedure, any of the compounds described above as embodiments A, B, or C or alternative embodiments A or B, can inhibit platelet binding to exposed collagen of the denuded endothelium, platelet activation, intimal hyperplasia, and/or vasospasm, or can bind to collagen in a denuded vessel.

In another illustrative embodiment, DS-SILY₁₈ can inhibit platelet binding to exposed collagen of the denuded endothelium, platelet activation, thrombosis, inflammation resulting from denuding the endothelium, intimal hyperplasia, and/or vasospasm, or can stimulate endothelial cell proliferation or can bind to collagen in a denuded vessel. In another illustrative embodiment, during a vascular intervention procedure, DS-SILY₁₈ can inhibit platelet binding to exposed collagen of the denuded endothelium, platelet activation, thrombosis, inflammation resulting from denuding the endothelium, intimal hyperplasia, and/or vasospasm, or can stimulate endothelial cell proliferation or can bind to collagen in a denuded vessel. In another illustrative embodiment, during a vascular intervention procedure, DS-SILY18 can inhibit platelet binding to exposed collagen of the denuded endothelium, platelet activation, intimal hyperplasia, and/or vasospasm, or can bind to collagen in a denuded vessel.

In various embodiments described herein, the synthetic peptides described herein can be modified by the inclusion of one or more conservative amino acid substitutions. As is well known to those skilled in the art, altering any non-critical amino acid of a peptide by conservative substitution should not significantly alter the activity of that peptide because the side-chain of the replacement amino acid should be able to form similar bonds and contacts to the side chain of the amino acid which has been replaced.

Non-conservative substitutions are possible provided that these do not excessively affect the collagen binding activity of the peptide and/or reduce its effectiveness in inhibiting platelet activation, platelet binding to exposed collagen of the denuded endothelium, platelet activation, thrombosis, inflammation resulting from denuding the endothelium, intimal hyperplasia, and/or vasospasm, or its effectiveness in stimulating endothelial cell proliferation or in binding to collagen in a denuded vessel.

As is well-known in the art, a "conservative substitution" of an amino acid or a "conservative substitution variant" of a peptide refers to an amino acid substitution which maintains: 1) the secondary structure of the peptide; 2) the charge or hydrophobicity of the amino acid; and 3) the bulkiness of the side chain or any one or more of these characteristics. Illustratively, the well-known terminologies "hydrophilic residues" relate to serine or threonine. "Hydrophobic residues" refer to leucine, isoleucine, phenylalanine, valine or alanine. "Positively charged residues" relate to lysine, arginine, omithine, or histidine. "Negatively charged residues" refer to aspartic acid or glutamic acid. Residues having "bulky side chains" refer to phenylalanine, tryptophan or tyrosine. A list of illustrative conservative amino acid substitutions is given in TABLE 1.

**TABLE 1**

| For Amino Acid | Replace With |
|---|---|
| Alanine | D-Ala, Gly, Aib, B-Ala, L-Cys, D-Cys |
| Arginine | D-Arg, Lys, D-Lys, Orn D-Om |
| Asparagine | D-Asn, Asp, D-Asp, Glu, D-Glu Gln, D-Gln |
| Aspartic Acid | D-Asp, D-Asn, Asn, Glu, D-Glu, Gln, D-Gln |
| Cysteine | D-Cys, S-Me-Cys, Met, D-Met, Thr, D-Thr |
| Glutamine | D-Gln, Asn, D-Asn, Glu, D-Glu, Asp, D-Asp |
| Glutamic Acid | D-Glu, D-Asp, Asp, Asn, D-Asn, Gln, D-Gln |
| Glycine | Ala, D-Ala, Pro, D-Pro, Aib, β-Ala |
| Isoleucine | D-Ile, Val, D-Val, Leu, D-Leu, Met, D-Met |
| Leucine | Val, D-Val, Met, D-Met, D-Ile, D-Leu, Ile |
| Lysine | D-Lys, Arg, D-Arg, Orn, D-Orn |
| Methionine | D-Met, S-Me-Cys, Ile, D-Ile, Leu, D-Leu, Val, D-Val |
| Phenylalanine | D-Phe, Tyr, D-Tyr, His, D-His, Trp, D-Trp |
| Proline | D-Pro |
| Serine | D-Ser, Thr, D-Thr, allo-Thr, L-Cys, D-Cys |
| Threonine | D-Thr, Ser, D-Ser, allo-Thr, Met, D-Met, Val, D-Val |
| Tyrosine | D-Tyr, Phe, D-Phe, His, D-His, Trp, D-Trp |
| Valine | D-Val, Leu, D-Leu, Ile, D-Ile, Met, D-Met |

In the various conservative amino acid substitution embodiments described herein, a collagen-binding synthetic peptidoglycan comprising a synthetic peptide of about 5 to about 40 amino acids with amino acid sequence homology to a collagen binding peptide (e.g. a portion of an amino acid sequence of a collagen binding protein or proteoglycan) conjugated to a glycan selected from the group consisting of alginate, agarose, dextran, chondroitin, dermatan, dermatan sulfate, heparan, heparin, keratin, and hyaluronan can be used. In one embodiment, the glycan is selected from the group consisting of dermatan sulfate, dextran, hyaluronan, and heparin. In another illustrative embodiment the glycan is dermatan sulfate. In yet another embodiment, the glycan is dermatan sulfate with 18 peptides of the sequence RRANAALKAGELYKSILYGC linked to the glycan and this sequence can be conservatively substituted. The collagen-binding synthetic proteoglycan in any of these conservative substitution embodiments can be used to inhibit platelet binding to exposed collagen of the denuded endothelium, platelet activation, thrombosis, inflammation resulting from denuding the endothelium, intimal hyperplasia, and/or vasospasm. The collagen-binding synthetic peptidoglycans described herein with conservative amino acid substitutions can also stimulate endothelial cell proliferation and can bind to collagen in a denuded vessel. In any of these embodiments, these aforementioned effects can occur during a vascular intervention procedure, such as a catheter-based procedure. In any of these conservative substitution embodiments, any of the above-described compounds can be used.

In another illustrative disclosure, any of the compounds selected from the group consisting of RRANAALKAGELYKSILYGC, RLDGNEIKRGC, AHEEISTTNEGVMGC, NGVFKYRPRYFLYKHAYFYPPLKRFPVQGC, CQDSETRTFY, TKKTLRTGC, GLRSKSKKFRRPDIQYPDATDEDITSHMGC, SQNPVQPGC, SYIRIADTNITGC, SYIRIADTNIT, KELNLVYT, KELNLVYTGC, GSITTIDVPWNV, GELYKSILYGC, GSITTIDVPWNVGC, and GCGGELYKSILY having conservative amino acid substitutions can be used. In any of these embodiments the compounds with conservative amino acid substitutions can inhibit platelet binding to exposed collagen of the denuded endothelium, platelet activation, thrombosis, inflammation resulting from denuding the endothelium, intima 1 hyperplasia, and/or vasospasm, or can stimulate endothelial cell proliferation or can bind to collagen in a denuded vessel. In another illustrative embodiment, during a vascular intervention procedure, any of these compounds with conservative amino acid substitutions can inhibit platelet binding to exposed collagen of the denuded endothelium, platelet activation, thrombosis, inflammation resulting from denuding the endothelium, intimal hyperplasia, and/or vasospasm, or can stimulate endothelial cell proliferation or can bind to collagen in a denuded vessel. In another illustrative embodiment, during a vascular intervention procedure, any of the compounds with conservative amino acid substitutions described in this paragraph can inhibit platelet binding to exposed collagen of the denuded endothelium, platelet activation, intimal hyperplasia, and/or vasospasm, or can bind to collagen in a denuded vessel.

In various embodiments described herein, the synthetic peptide is synthesized according to solid phase peptide synthesis protocols that are well known by persons of skill in the art. In one embodiment a peptide precursor is synthesized on a solid support according to the well-known Fmoc protocol, cleaved from the support with trifluoroacetic acid and purified by chromatography according to methods known to persons skilled in the art.

In various embodiments described herein, the synthetic peptide is synthesized utilizing the methods of biotechnology that are well known to persons skilled in the art. In one embodiment a DNA sequence that encodes the amino acid sequence information for the desired peptide is ligated by recombinant DNA techniques known to persons skilled in the art into an expression plasmid (for example, a plasmid that incorporates an affinity tag for affinity purification of the peptide), the plasmid is transfected into a host organism for expression, and the peptide is then isolated from the host organism or the growth medium according to methods known by persons skilled in the art (e.g., by affinity purification). Recombinant DNA technology methods are described in Sambrook et al., "Molecular Cloning: A Laboratory Manual", 3rd Edition, Cold Spring Harbor Laboratory Press, (2001), and are well-known to the skilled artisan.

In various embodiments described herein, the synthetic peptide is conjugated to a glycan by reacting a free amino group of the peptide with an aldehyde function of the glycan in the presence of a reducing agent, utilizing methods known to persons skilled in the art, to yield the peptide glycan conjugate. In one embodiment an aldehyde function of the glycan (e.g. polysaccharide or glycosaminoglycan) is formed by reacting the glycan with sodium metaperiodate according to methods known to persons skilled in the art.

In one embodiment, the synthetic peptide is conjugated to a glycan by reacting an aldehyde function of the glycan with 3-(2-pyridyldithio) propionyl hydrazide (PDPH) to form an intermediate glycan and further reacting the intermediate glycan with a peptide containing a free thiol group to yield the peptide glycan conjugate. In yet another embodiment, the sequence of the peptide may be modified to include a glycine-cysteine segment to provide an attachment point for a glycan or a glycan-linker conjugate.

In any of the embodiments described herein, the synthetic peptide is conjugated to a glycan by reacting an aldehyde function of the glycan with a crosslinker, e.g., 3-(2-pyridyldithio)propionyl hydrazide (PDPH), to form an intermediate glycan and further reacting the intermediate glycan with a peptide containing a free thiol group to yield the peptide glycan conjugate. In any of the various embodiments described herein, the sequence of the peptide may be modified to include a glycine-cysteine segment to provide an attachment point for a glycan or a glycan-linker conjugate. In any of the embodiments described herein, the crosslinker can be N-[β-Maleimidopropionic acid]hydrazide (BMPH).

Although specific embodiments have been described in the preceding paragraphs, the collagen-binding synthetic peptidoglycans described herein can be made by using any art-recognized method for conjugation of the peptide to the glycan (e.g. polysaccharide or glycosaminoglycan). This can include covalent, ionic, or hydrogen bonding, either directly or indirectly via a linking group such as a divalent linker. The conjugate is typically formed by covalent bonding of the peptide to the glycan through the formation of amide, ester or imino bonds between acid, aldehyde, hydroxy, amino, or hydrazo groups on the respective components of the conjugate. All of these methods are known in the art or are further described in the Examples section of this application or in Hermanson G.T., Bioconjugate Techniques, Academic Press, pp.169-186 (1996). The linker typically comprises about 1 to about 30 carbon atoms, more typically about 2 to about 20 carbon atoms. Lower molecular weight linkers (i.e., those having an approximate molecular weight of about 20 to about 500) are typically employed.

In addition, structural modifications of the linker portion of the conjugates are contemplated herein. For example, amino acids may be included in the linker and a number of amino acid substitutions may be made to the linker portion of the conjugate, including naturally occurring amino acids, as well as those available from conventional synthetic methods. In another aspect, beta, gamma, and longer chain amino acids may be used in place of one or more alpha amino acids. In another aspect, the linker may be shortened or lengthened, either by changing the number of amino acids included therein, or by including more or fewer beta, gamma, or longer chain amino acids. Similarly, the length and shape of other chemical fragments of the linkers described herein may be modified.

In various embodiments described herein, the linker may include one or more bivalent fragments selected independently in each instance from the group consisting of alkylene, heteroalkylene, cycloalkylene, cycloheteroalkylene, arylene, and heteroarylene each of which is optionally substituted. As used herein heteroalkylene represents a group resulting from the replacement of one or more carbon atoms in a linear or branched alkylene group with an atom independently selected in each instance from the group consisting of oxygen, nitrogen, phosphorus and sulfur.

In various embodiments described herein, a collagen-binding synthetic peptidoglycan may be administered to a patient (e.g., a patient in need of treatment to inhibit platelet activation, such as that involved in thrombosis, platelet binding to exposed collagen of the denuded endothelium, thrombosis, inflammation resulting from denuding the endothelium, intimal hyperplasia, or vasospasm). In various embodiments, the collagen-binding synthetic peptidoglycan can be administered intravenously or into muscle, for example. Suitable routes for parenteral administration include intravascular, intravenous, intraarterial, intramuscular, cutaneous, subcutaneous, percutaneous, intradermal, and intraepidermal delivery. Suitable means for parenteral administration include needle (including microneedle) injectors, infusion techniques, and catheter-based delivery.

In an illustrative embodiment, pharmaceutical formulations for use with collagen-binding synthetic peptidoglycans for parenteral administration or catheter-based delivery comprising: a) a pharmaceutically active amount of the collagen-binding synthetic peptidoglycan; b) a pharmaceutically acceptable pH buffering agent to provide a pH in the range of about pH 4.5 to about pH 9; c) an ionic strength modifying agent in the concentration range of about Oto about 300 millimolar; and d) water soluble viscosity modifying agent in the concentration range of about 0.25% to about 10% total formula weight or any individual component a), b), c), or d) or any combinations of a), b), c) and d) are provided.

In various embodiments described herein, the pH buffering agents for use in the compositions and methods herein described are those agents known to the skilled artisan and include, for example, acetate, borate, carbonate, citrate, and phosphate buffers, as well as hydrochloric acid, sodium hydroxide, magnesium oxide, monopotassium phosphate, bicarbonate, ammonia, carbonic acid, hydrochloric acid, sodium citrate, citric acid, acetic acid, disodium hydrogen phosphate, borax, boric acid, sodium hydroxide, diethyl barbituric acid, and proteins, as well as various biological buffers, for example, TAPS, Bicine, Tris, Tricine, HEPES, TES, MOPS, PIPES, cacodylate, or MES.

In various embodiments described herein, the ionic strength modifying agents include those agents known in the art, for example, glycerin, propylene glycol, mannitol, glucose, dextrose, sorbitol, sodium chloride, potassium chloride, and other electrolytes.

Useful viscosity modulating agents include ionic and non- ionic water soluble polymers; crosslinked acrylic acid polymers such as the "carbomer" family of polymers, e.g., carboxypolyalkylenes that may be obtained commercially under the Carbopol® trademark; hydrophilic polymers such as polyethylene oxides, polyoxyethylene- polyoxypropylene copolymers, and polyvinylalcohol; cellulosic polymers and cellulosic polymer derivatives such as hydroxypropyl cellulose, hydroxyethyl cellulose, hydroxypropyl methylcellulose, hydroxypropyl methylcellulose phthalate, methyl cellulose, carboxymethyl cellulose, and etherified cellulose; gums such as tragacanth and xanthan gum; sodium alginate; gelatin, hyaluronic acid and salts thereof, chitosans, gellans or any combination thereof.

Typically, non-acidic viscosity enhancing agents, such as a neutral or basic agent are employed in order to facilitate achieving the desired pH of the formulation.

In various embodiments described herein, parenteral formulations may be suitably formulated as a sterile non-aqueous solution or as a dried form to be used in conjunction with a suitable vehicle such as sterile, pyrogen-free water. The preparation of
parenteral formulations under sterile conditions, for example, by lyophilisation, may readily be accomplished using standard pharmaceutical techniques well known to those skilled in the art.

In various embodiments described herein, the solubility of a collagen-binding synthetic peptidoglycan used in the preparation of a parenteral formulation may be increased by the use of appropriate formulation techniques, such as the incorporation of solubility-enhancing compositions such as mannitol, ethanol, glycerin, polyethylene glycols, propylene glycol, poloxomers, and others known to those of skill in the art.

In various embodiments described herein, formulations for parenteral administration may be formulated to be for immediate and/or modified release. Modified release formulations include delayed, sustained, pulsed, controlled, targeted and programmed release formulations. Thus, a collagen-binding synthetic peptidoglycan may be formulated as a solid, semi-solid, or thixotropic liquid for administration as an implanted depot providing modified release of the active compound. Illustrative examples of such formulations include drug-coated stents and copolymeric(dl-lactic, glycolic)acid (PGLA) microspheres. In another embodiment, collagen-binding synthetic peptidoglycans or compositions comprising collagen-binding synthetic peptidoglycan may be continuously administered, where appropriate.

In any of the embodiments described herein, the collagen-binding synthetic peptidoglycan can be administered intravascularly into the patient (e.g., into an artery or vein) in any suitable way. In various embodiments described herein, the collagen-binding synthetic peptidoglycan can be administered into a vessel of a patient prior to, during, or after vascular intervention. In various embodiments, vascular interventions, such as percutaneous coronary intervention (PCI), can include, for example, stenting, atherectomy, grafting, and angioplasty, such as balloon angioplasty. Illustratively, the vascular intervention can be one which involves temporarily occluding an artery, such as a coronary artery or a vein (e.g., balloon angioplasty), or it can be one which does not involve temporarily occluding an artery or a vein (e.g., non-balloon angioplasty procedures, stenting procedures that do not involve balloon angioplasty, etc.). Illustrative modes of delivery can include a catheter, parenteral administration, a coating on a ballon, through a porous ballon, a coated stent, and any combinations thereof or any other known methods of delivery of drugs during a vascular intervention procedure. In one illustrative embodiment, the target vessel can include a coronary artery, e.g., any blood vessel which supplies blood to the heart tissue of a patient, including native coronary arteries as well as those which have been grafted into the patient, for example, in an earlier coronary artery bypass procedure.

In any of the embodiments described herein, the target vessel into which the collagen-binding synthetic peptidoglycan is to be administered and on which the vascular intervention procedure is to be performed may contain a blockage, such as a stenosis or some other form of complete or partial blockage which causes reduced blood flow through the vessel. Thus, the collagen-binding synthetic peptidoglycan can be delivered to the vessel via a catheter (e.g., a dilatation catheter, an over-the-wire angioplasty balloon catheter, an infusion catheter, a rapid exchange or monorail catheter, or any other catheter device known in the art) which is percutaneously inserted into the patient and which is threaded through the patient's blood vessels to the target vessel. Various catheter-based devices are known in the art, including those described in U.S. Patent No. 7,300,454. In various embodiments described herein where a catheter is used, the catheter used to deliver the collagen-binding synthetic peptidoglycan can be the same catheter through which the vascular intervention is to be performed, or it can be a different catheter (e.g., a different catheter which is percutaneously inserted into the patient via the same or a different cutaneous incision and/or which is threaded through the patient's blood vessels to the target vessel via the same or a different route). In another embodiment, the collagen-binding synthetic peptidoglycan can be injected directly into the target vessel. In another embodiment, the collagen-binding synthetic peptidoglycan can be delivered systemically (i.e., not delivered directly to the target vessel, but delivered by parenteral administration without catheter-based delivery).

In the case where the vessel contains a blockage (e.g., a stenosis), administration can be carried out by delivering the collagen-binding synthetic peptidoglycan directly to the target vessel at the site of the blockage or distal to the blockage or both. In another embodiment, the collagen-binding synthetic peptidoglycan can be delivered to one or more sites proximal to the blockage. Illustratively, the catheter tip can be maintained stationary while the collagen-binding synthetic peptidoglycan is being delivered, or the catheter tip can be moved while the collagen-binding synthetic peptidoglycan is being delivered (e.g., in a proximal direction from a position that is initially distal to the blockage, to or through the blockage, or to a position which is proximal to the blockage).

As indicated above, in one embodiment, the collagen-binding synthetic peptidoglycan can be administered directly into the patient's vessel at a time prior to vascular intervention, e.g., percutaneous coronary intervention. For example, delivery of the collagen- binding synthetic peptidoglycan can be carried out just prior to vascular intervention (e.g., within about 1 hour, such as within about 30 minutes, within about 15 minutes, and/or within
about 5 minutes prior to vascular intervention). Optionally, delivery of the collagen-binding synthetic peptidoglycan directly to the target vessel can be continued during all or part of the vascular intervention procedure and/or subsequent to completion of such procedure, or delivery of the collagen-binding synthetic peptidoglycan directly to the target vessel can be stopped prior to the commencement of the vascular intervention procedure and not subsequently recommenced. In any of the embodiments described herein, delivery of the collagen-binding synthetic peptidoglycan can be continuous or it can be effected through a single or multiple administrations. Prior to, during, and/or after the collagen-binding synthetic peptidoglycan is administered to the target vessel, the same collagen-binding synthetic peptidoglycan or one or more different collagen-binding synthetic peptidoglycans can be administered.

In any of the embodiments described herein, the collagen-binding synthetic peptidoglycan can be administered alone or in combination with suitable pharmaceutical carriers or diluents. Diluent or carrier ingredients used in the collagen-binding synthetic peptidoglycan formulation can be selected so that they do not diminish the desired effects of the collagen-binding synthetic peptidoglycan. The collagen-binding synthetic peptidoglycan formulation may be in any suitable form. Examples of suitable dosage forms include aqueous solutions of the collagen-binding peptidoglycan, for example, a solution in isotonic saline, 5% glucose or other well-known pharmaceutically acceptable liquid carriers such as alcohols, glycols, esters and amides.

Suitable dosages of the collagen-binding synthetic peptidoglycan can be determined by standard methods, for example by establishing dose-response curves in laboratory animal models or in clinical trials. Illustratively, suitable dosages of collagen-binding synthetic peptidoglycan (administered in a single bolus or over time) include from 1 ng/kg to about 10 mg/kg, 100 ng/kg to about 1 mg/kg, from about 1 µg/kg to about 500 µg/kg, or from about 100 µg/kg to about 400 µg/kg. In each of these embodiments, dose/kg refers to the dose per kilogram of patient mass or body weight. In other illustrative aspects, effective doses can range from about 0.01 µg to about 1000 mg per dose, 1 µg to about 100 mg per dose, or from about 100 µg to about 50 mg per dose, or from about 500 µg to about 10 mg per dose or from about 1 mg to 10 mg per dose, or from about 1 to about 100 mg per dose, or from about 1 mg to 5000 mg per dose, or from about 1 mg to 3000 mg per dose, or from about 100 mg to 3000 mg per dose, or from about 1000 mg to 3000 mg per dose.

Vascular intervention, such as percutaneous coronary intervention, can be carried out by any conventional procedure prior to, during, or after administration of the collagen-binding synthetic peptidoglycan. Examples of vascular intervention procedures contemplated for use in conjunction with the method of the present invention include stenting, atherectomy, and angioplasty, such as balloon angioplasty. The vascular intervention procedure can be one which involves temporarily occluding the vessel (e.g., balloon angioplasty), or it can be one which does not involve temporarily occluding the vessel (e.g., non-balloon angioplasty procedures, stenting procedures that do not involve balloon angioplasty, etc.). Illustrative modes of delivery can include a catheter, parenteral administration, a coating on a ballon, through a porous ballon, a coated stent, and any combinations thereof or any other known methods of delivery of drugs during a vascular intervention procedure.

In any of the embodiments herein described, kits for carrying out vascular intervention, such as the kits described above, are contemplated. The kits can include a catheter or a stent and a collagen-binding synthetic peptidoglycan. The collagen-binding synthetic peptidoglycan can be provided in any of the formulations discussed above and in an amount needed to carry our a single vascular intervention, such as from 1 ng/kg to about 10 mg/kg, 100 ng/kg to about 1 mg/kg, from about 1 µg/kg to about 500 µg/kg, or from about 100 µg/kg to about 400 µg/kg. In each of these embodiments, dose/kg refers to the dose per kilogram of patient mass or body weight. In various embodiments herein described, effective doses provided in the formulations can range from about 0.01 µg to about 1000 mg per dose, 1 µg to about 100 mg per dose, or from about 100 µg to about 50 mg per dose, or from about 500 µg to about 10 mg per dose or from about 1 mg to 10 mg per dose, or from about 1 to about 100 mg per dose, or from about 1 mg to 5000 mg per dose, or from about 1 mg to 3000 mg per dose, or from about 100 mg to 3000 mg per dose, or from about 1000 mg to 3000 mg per dose. Articles of manufacture are also contemplated for any of these embodiments.

In any of the kit or article of manufacture embodiments described herein, the kit or article of manufacture can comprise a dose or multiple doses of the collagen-binding synthetic peptidoglycan. The collagen-binding synthetic peptidoglycan can be in a primary container, for example, a glass vial, such as an amber glass vial with a rubber stopper and/or an aluminum tear-off seal. In another embodiment, the primary container can be plastic or aluminum, and the primary container can be sealed. In another embodiment, the primary container may be contained within a secondary container to further protect the composition from light.

In any of the embodiments described herein, the kit or article of manufacture can contain instructions for use. Other suitable kit or article of manufacture components include excipients, disintegrants, binders, salts, local anesthetics (*e.g*., lidocaine), diluents, preservatives, chelating agents, buffers, tonicity agents, antiseptic agents, wetting agents, emulsifiers, dispersants, stabilizers. These components may be available separately or admixed with the collagen-binding synthetic peptidoglycan. Any of the composition embodiments described herein can be used to formulate the kit or article of manufacture.

In various embodiments herein described, the kit can contain more than one catheter or a stent and a plurality of separate containers, each containing sterilized collagen- binding synthetic peptidoglycan formulations in an amount needed to carry out a single or multiple vascular interventions. Any type of stent or catheter may be included with the kit, including, for example, dilatation catheters, over-the-wire angioplasty balloon catheters, infusion catheters, rapid exchange or monorail catheters.

It is also contemplated that any of the formulations described herein may be used to administer the collagen-binding synthetic peptidoglycan (e.g., one or more types) either in the absence or the presence of a catheter-based device. The collagen-binding synthetic proteoglycan can be formulated in an excipient. In any of the embodiments described herein, the excipient can have a concentration ranging from about 0.4 mg/ml to about 6 mg/ml. In various embodiments, the concentration of the excipient may range from about 0.5 mg/ml to about 10 mg/ml, about 0.1 mg/ml to about 6 mg/ml, about 0.5 mg/ml to about 3 mg/ml, about 1 mg/ml to about 3 mg/ml, about 0.01 mg/ml to about 10 mg/ml, and about 2 mg/ml to about 4 mg/ml.

In various embodiments described herein, the dosage of the collagen-binding synthetic peptidoglycan, can vary significantly depending on the patient condition, the disease state being treated, the route of administration and tissue distribution, and the possibility of co-usage of other therapeutic treatments. The effective amount to be administered to a patient is based on body surface area, patient weight or mass, and physician assessment of patient condition. In various exemplary embodiments, an effective dose can range from about 1 ng/kg to about 10 mg/kg, 100 ng/kg to about 1 mg/kg, from about 1 µg/kg to about 500 µg/kg, or from about 100 µg/kg to about 400 µg/kg. In each of these embodiments, dose/kg refers to the dose per kilogram of patient mass or body weight. In other illustrative aspects, effective doses can range from about 0.01 µg to about 1000 mg per dose, 1 µg to about 100 mg per dose, or
from about 100 µg to about 50 mg per dose, or from about 500 µg to about 10 mg per dose or from about 1 mg to 10 mg per dose, or from about 1 to about 100 mg per dose, or from about 1 mg to 5000 mg per dose, or from about 1 mg to 3000 mg per dose, or from about 100 mg to 3000 mg per dose, or from about 1000 mg to 3000 mg per dose. In any of the various embodiments described herein, effective doses can range from about 0.01 µg to about 1000 mg per dose, 1 µg to about 100 mg per dose, about 100 µg to about 1.0 mg, about 50 µg to about 600 µg, about 50 µg to about 700 µg, about 100 µg to about 200 µg, about 100 µg to about 600 µg, about 100 µg to about 500 µg, about 200 µg to about 600µg, or from about 100 µg to about 50 mg per dose, or from about 500 µg to about 10 mg per dose or from about 1 mg to 10 mg per dose. In other illustrative embodiments, effective doses can be 1 µg, 10 µg, 25 µg, 50µg, 75 µg, 100 µg, 125 µg, 150µg, 200 µg, 250 µg, 275 µg, 300 µg, 350µg, 400µg, 450µg, 500 µg, 550 µg, 575 µg, 600 µg, 625 µg, 650 µg, 675 µg, 700µg, 800 µg, 900µg, 1.0 mg, 1.5 mg, 2.0 mg, 10 mg, 100 mg, or 100 mg to 30 grams.

Any effective regimen for administering the collagen-binding synthetic peptidoglycan can be used. For example, the collagen-binding synthetic peptidoglycan can be administered as a single dose, or as a multiple-dose daily regimen. Further, a staggered regimen, for example, one to five days per week can be used as an alternative to daily treatment.

In various embodiments described herein, the patient is treated with multiple injections of the collagen-binding synthetic peptidoglycan. In one embodiment, the patient is injected multiple times (e.g., about 2 up to about 50 times) with the collagen-binding synthetic peptidoglycan, for example, at 12-72 hour intervals or at 48-72 hour intervals. Additional injections of the collagen-binding synthetic peptidoglycan can be administered to the patient at an interval of days or months after the initial injections(s).

In any of the embodiments herein described, it is to be understood that a combination of two or more collagen-binding synthetic peptidoglycans, differing in the peptide portion, the glycan portion, or both, can be used in place of a single collagen-binding synthetic peptidoglycan.

It is also appreciated that in the foregoing embodiments, certain aspects of the compounds, compositions and methods are presented in the alternative in lists, such as, illustratively, selections for any one or more of G and P. It is therefore to be understood that various alternate embodiments of the invention include individual members of those lists, as well as the various subsets of those lists. Each of those combinations are to be understood to be described herein by way of the lists.

In the following illustrative examples, the terms "synthetic peptidoglycan" and "conjugate" are used synonymously with the term "collagen-binding synthetic peptidoglycan."

### EXAMPLE 1

### Peptide Synthesis

All peptides were synthesized using a Symphony peptide synthesizer (Protein Technologies, Tucson, AZ), utililizing an FMOC protocol on a Knorr resin. The crude peptide was released from the resin with TFA and purified by reverse phase chromatography on an AKTAexplorer (GE Healthcare, Piscataway, NJ) utililizing a Grace-Vydac 218TP C-18 reverse phase column and a gradient of water/acetonitrile 0.1 %TFA. Dansyl-modified peptides were prepared by adding an additional coupling step with dansyl-Gly (Sigma) before release from the resin. Peptide structures were confirmed by mass spectrometry. The following peptides were prepared as described above: RRANAALKAGELYKSILYGC, SYIRIADTNIT, Dansyl-GRRANAALKAGELYKSILYGC, and Dansyl-GSYIRIADTNIT. These peptides are abbreviated SILY, SYIR, Z-SILY, and Z-SYIR. A biotin-labeled Z-SYIR peptide has also been synthesized using protocols known in the art and the peptide is amide terminated. Additional peptides, KELNLVYTGC (abbreviated KELN) and GSITTIDVPWNVGC (abbreviated GSIT) were prepared as described above or purchased (GenScript, Piscataway, NJ).

### EXAMPLE 2

### Conjugation of SILY to Dermatan Sulfate

### PDPH Attachment to oxDS

The bifunctional crosslinker PDPH (Pierce), reactive to sulfhydryl and amine groups, was used to conjugate SILY to oxDS. In the first step of the reaction, oxDS was dissolved in coupling buffer (0.1 M sodium phosphate, 0.25 M sodium chloride, pH 7.2) to a final concentration of 1.2 mM. PDPH was added in 10-fold molar excess, and the reaction proceeded at room temperature for 2 hours. Excess PDPH (MW 229 Da) was separated by gel filtration on an Akta Purifier using an XK 26-40 column packed with Sephadex G-25 medium and equilibrated with MilliQ water. Eluent was monitored at 215 nm, 254 nm, and 280 nm. The first eluting peak containing DS-PDPH was collected and lyophilized for conjugating with SILY.

### Conjugation of SILY

The peptide was dissolved in a 5: 1 molar excess in coupling buffer at a final peptide concentration of approximately 1 mM (limited by peptide solubility). The reaction was allowed to proceed at room temperature overnight, and excess peptide was separated and the DS-SILY conjugate isolated by gel filtration as described above. See FIGURE 14 showing a SILY/DS ratio of 1.06 after coupling.

### EXAMPLE 3

### Conjugation of Z-SILY to Dermatan Sulfate

Dermatan sulfate was conjugated to Z-SILY according to the method of EXAMPLE 2.

### REFERENCE EXAMPLE 4

### Conjugation of KELN to Dermatan Sulfate

Dermatan sulfate was conjugated to KELN according to the method of EXAMPLE 2.

### REFERENCE EXAMPLE 5

### Conjugation of GSIT to Dermatan Sulfate

Dermatan sulfate was conjugated to GSIT according to the method of EXAMPLE 2.

### REFERENCE EXAMPLE 6

### Conjugation of Z-SYIR to Dermatan Sulfate

Dermatan sulfate was conjugated to Z-SYIR using a method similar to that described in EXAMPLE 2.

### EXAMPLE 7

### Conjugation of SILY to Heparin

Oxidized Heparin (oxHep) (MW = 19.7kDa) containing 1 aldehyde per molecule (purchased from Celsus Laboratories, Cincinnati, OH). Additional aldehydes were formed by further oxidation in sodium meta-periodate as follows. oxHep was dissolved in 0.1 M sodium acetate pH 5.5 at a concentration of 10 mg/mL. Sodium meta-periodate was then added at a concentration of 2 mg/mL and allowed to react for 4 hours at room temperature protected from light. Excess sodium meta-periodate was removed by desalting using a HiTrap size exclusion column (GEHealthcare) and oxHep was lyophilized protected from light until conjugation with PDPH.

oxHep was conjugated to PDPH by the method described for DS-PDPH conjugation, EXAMPLE2. PDPH was reacted in 50-fold molar excess. To achieve a higher PDPH concentration, 10 mg PDPH was dissolved in 75 µL DMSO and mixed with 1 mL coupling buffer containing oxHep. The reaction proceeded at room temperature for 2.5 hours and excess PDPH was removed by desalting. Heparin containing PDPH (Hep-PDPH) was stored as a lyophilized powder until reacted with SILY.

SILY was reacted in 10-fold molar excess with Hep-PDPH as described for DS- SILY conjugation in EXAMPLE2. The reaction was monitored as described for DS-SILY in EXAMPLE2 and showed 5.44 SILY peptides conjugated per heparin molecule as shown in FIGURE 13.

### REFERENCE EXAMPLE 8

### Conjugation of GSIT to Heparin

Heparin was conjugated to GSIT according to the method of EXAMPLE7 (abbreviated Hep-GSIT).

### EXAMPLE 9

### Conjugation of SILY to Dextran

Dextran was conjugated to SILY according to the method of EXAMPLE7 replacing heparin with dextran. Modification of the conditions for oxidation of dextran with sodium meta-periodate in the first step to allowed preparation of conjugates with different molar ratios of SILY to dextran. For example dextran-SILY conjugates with a molar ratio of SILY to dextran of about 6 and a dextran-SILY conjugate with a molar ratio of SILY to dextran of about 9 were prepared (abbreviated Dex-SILY6 and Dex-SILY9).

### EXAMPLE 10

### Conjugation of SILY to Hyaluronan

Hyaluronan was conjugated to SILY according to the method of EXAMPLE7 (abbreviated HA-SILY).

### EXAMPLE 11

### SILY Binding to Collagen (Biacore)

Biacore studies were performed on a Biacore 2000 using a CM-3 chip (Biacore, Inc., Piscataway, NJ). The CM-3 chip is coated with covalently attached carboxymethylated dextran, which allows for attachment of the substrate collagen via free amine groups. Flow cells (FCs) 1 and 2 were used, with FC-1 as the reference cell and FC-2 as the collagen immobilized cell. Each FC was activated with EDC-NHS, and 1500 RU of collagen was immobilized on FC-2 by flowing 1 mg/mL collagen in sodium acetate, pH 4, buffer at 5 µL/min for 10 min. Unreacted NHS-ester sites were capped with ethanolamine; the control FC-1 was activated and capped with ethanolamin.

To determine peptide binding affinity, SILY was dissolved in 1x HBS-EP buffer (Biacore) at varying concentrations from 100 µM to 1.5 µm in 2-fold dilutions. The flow rate was held at 90 µL/min which is in the range suggested by Myska for determining binding kinetics (Myska, 1997). The first 10 injections were buffer injections, which help to prime the system, followed by randomized sample injections, run in triplicate. Analysis was performed using BIAevaluation software (Biacore). Representative association/disassociation curves are shown in FIGURE 3 demonstrating that the SILY peptide binds reversibly with collagen. K_{D}=1.2 µM was calculated from the on-off binding kinetics.

### EXAMPLE 12

### Z-SILY Binding to Collagen

Binding assays were done in a 96-well high-binding plate, black with a clear bottom (Costar). Collagen was compared to untreated wells and BSA coated wells. Collagen and BSA were immobilized at 37°C for 1 hr by incubating 90 µL/well at concentrations of 2 mg/mL in 10 mM HCl and 1xPBS, respectively. Each well was washed 3x with 1xPBS after incubating. Z-SILY was dissolved in 1xPBS at concentrations from 100 µM to 10 nM in 10-fold dilutions. Wells were incubated for 30 min at 37°C and rinsed 3X with PBS and then filled with 90 µL of 1xPBS. Fluorescence readings were taken on an M5 Spectramax Spectrophotometer (Molecular Devices) at excitation/emission wavelengths of 335 nm/490 nm respectively. The results are shown in FIGURES 4 and 5. K_{D}=0.86 µM was calculated from the equilibrium kinetics.

### EXAMPLE 13

### Charaterizing DS-SILY

To determine the number of SILY molecules conjugated to DS, the production of pyridine-2-thione was measured using a modified protocol provided by Pierce. Dermatan sulfate with 1.1 PDPH molecules attached was dissolved in coupling buffer (0.1 M sodium phosphate, 0.25 M sodium chloride) at a concentration of 0.44 mg/mL and absorbance at 343 nm was measured using a SpectraMax M5 (Molecular Devices). SILY was reacted in 5-fold molar excess and absorbance measurements were repeated immediately after addition of SILY and after allowing to react for 2 hours. To be sure SILY does not itself absorb at 343 nm, coupling buffer containing 0.15 mg/mL SILY was measured and was compared to absorbance of buffer alone.

The number of SILY molecules conjugated to DS was calculated by the extinction coefficient of pyridine-2-thione using the following equation (Abs₃₄₃/8080) X (MW_{Ds}/DS_{mg/mL}). The results are shown in FIGURE 14.

### EXAMPLE 14

### Collagen Binding, Fluorescence Data - DS-SILY

In order to determine whether the peptide conjugate maintained its ability to bind to collagen after its conjugation to DS, a fluorescent binding assay was performed. A fluorescently labeled version of SILY, Z-SILY, was synthesized by adding dansylglycine to the amine terminus. This peptide was conjugated to DS and purified using the same methods described for SILY.

Binding assays were done in a 96-well high binding plate, black with a clear bottom (Costar). Collagen was compared to untreated wells and BSA coated wells. Monomeric collagen (Advanced Biomatrix Cat. No. 5010) and BSA were immobilized at 37°C for 1 hr by incubating 90 µL/well at concentrations of 2 mg/mL in 10 mM HCl and 1xPBS respectively. Each well was washed 3x with 1xPBS after incubating.

Wells were preincubated with DS at 37°C for 30 min to eliminate nonspecific binding of DS to collagen. Wells were rinsed 3x with 1xPBS before incubating with DS-Z-SILY. DS-Z-SILY was dissolved in 1xPBS at concentrations from 100 µM to 10 nM in 10-fold dilutions. Wells were incubated for 30 min at 37°C and rinsed 3x and then filled with 90 µL of 1xPBS. Fluorescence readings were taken on an M5 Spectramax Spectrophotometer (Molecular Devices) at excitation/emission wavelengths of 335 nm/490 nm, respectively.

Fluorescence binding of DS-Z-SILY on immobilized collagen, BSA, and untreated wells are compared in FIGURE 7. Results show that DS-Z-SILY binds specifically to the collagen-treated wells over BSA and untreated wells. The untreated wells of the high bind plate were designed to be a positive control, though little binding was observed relative to collagen treated wells. These results suggest that SILY maintains its ability to bind to collagen after it is conjugated to DS. Preincubating with DS did not prevent binding, suggesting that the conjugate binds separately from DS alone.

### EXAMPLE 15

### Preparation Of Type I Collagen Gels

Gels were made with Nutragen collagen (Inamed, Freemont, CA) at a final concentration of 4 mg/mL collagen. Nutragen stock is 6.4 mg/mL in 10mM HCl. Gel preparation was performed on ice, and fresh samples were made before each test. The collagen solution was adjusted to physiologic pH and salt concentration, by adding appropriate volumes of 10x PBS (phosphate buffered saline), 1xPBS, and 1 M NaOH. For most experiments, samples of DS, decorin, DS-SILY, or DS-SYIR were added at a 10:1 collagen:sample molar ratio by a final 1xPBS addition (equal volumes across treatments) in which the test samples were dissolved at appropriate concentrations. In this way, samples are constantly kept at pH 7.4 and physiologic salt concentration. Collagen-alone samples received a 1xPBS addition with no sample dissolved. Fibrillogenesis will be induced by incubating neutralized collagen solutions at 37°C overnight in a humidified chamber to avoid dehydration. Gel solutions with collagen:sample molar ratios of other than 10:1 were prepared similarly.

### EXAMPLE 16

### Viscoelastic Characterization of Collagen Type III Containing Gels

Gels containing type III collagen were prepared as in EXAMPLE 15 with the following modifications: treated and untreated gel solutions were prepared using a collagen concentration of 1.5 mg/mL (90% collagen type III (Millipore), 10% collagen type I), 200 µL samples were pipetted onto 20 mm diameter wettable surfaces of hydrophobic printed slides. These solutions were allowed to gel at 37°C for 24 hours. Gels were formed from collagen alone, collagen treated with dermatan sulfate (1:1 and 5:1 molar ratio), and collagen treated with the collagen III-binding peptides alone (GSIT and KELN, 5:1 molar ratio) served as controls. The treated gels contained the peptidoglycans (DS-GSIT or DS-KELN at 1:1 and 5:1 molar ratios. All ratios are collagen:treatment compound ratios. The gels were characterized as in EXAMPLE 18, except the samples were tested over a frequency range from 0.1 Hz to 1.0 Hz at a controlled stress of 1.0 Pa. As shown in FIGURES 8 and 9, the dermatan sulfate-GSIT conjugate and the dermatan sulfate-KELN conjugate (synthetic peptidoglycans) can influence the viscoelastic properties of gels formed with collagen type III.

### EXAMPLE 17

### Fibrillogenesis

Collagen fibrillogenesis was monitored by measuring turbidity related absorbance at 313 nm providing information on rate of fibrillogenesis and fibril diameter. Gel solutions were prepared as described in EXAMPLE 15 (4 mg/mL collagen, 10:1 collagen:treatment, unless otherwise indicated) and 50 uL/well were added at 4° C to a 384- well plate. The plate was kept at 4°C for 4 hours before initiating fibril formation. A SpectraMax M5 at 37° C was used to measure absorbance at 313 nm at 30 s intervals for 6 hours. The results are shown in FIGURE 10. Dermatan sulfate-SILY decreases the rate of fibrillogenesis.

### EXAMPLE 18

### Cryo-SEM Measurements on Collagen Type III

Gels for cryo-SEM were formed, as in EXAMPLE 15, directly on the SEM stage and incubated at 37° overnight with the following modifications. The collagen concentration was 1 mg/mL (90% collagen type III, 10% collagen type I). The collagen:DS ratio was 1:1 and the collagen:peptidoglycan ratio was 1:1. The images were recorded as in EXAMPLE 19. The ratio of void volume to fibril volume was measured using a variation of the method in EXAMPLE 28. The results are shown in FIGURES 11 and 12. Dermatan sulfate-KELN and dermatan sulfate-GSIT decrease void space (increase fibril diameter and branching) in the treated collagen gels.

### EXAMPLE 19

### AFM Confirmation Of D-Banding

Gel solutions were prepared as described in EXAMPLE 15 and 20 µL of each sample were pipetted onto a glass coverslip and allowed to gel overnight in a humidified incubator. Gels were dehydrated by treatment with graded ethanol solutions (35%, 70%, 85%, 95%, 100%), 10 min in each solution. AFM images were made in contact mode, with a scan rate of 2 Hz (Multimode SPM, Veeco Instruments, Santa Barbara, CA, USA, AFM tips Silicon Nitride contact mode tip k=0.05N/m, Veeco Instruments) Deflection setpoint: 0-1 Volts. D- banding was confirmed in all treatments as shown in FIGURES 2 and 26.

### EXAMPLE 20

### Collagen Remodeling

### Tissue Sample Preparation

Following a method by Grassl, et al. (Grassl, et al., Journal of Biomedical Materials Research 2002, 60, (4), 607-612), collagen gels with or without synthetic PG mimics were formed as described in EXAMPLE 15. Human aortic smooth muscle cells (Cascade Biologies, Portland, OR) were seeded within collagen gels by adding 4 x 10⁶ cells/mL to the neutralized collagen solution prior to incubation. The cell-collagen solutions were pipetted into an 8-well Lab-Tek chamber slide and incubated in a humidified 37°C and 5% C02 incubator. After gelation, the cell-collagen gels will be covered with 1 mL Medium 231 as prescribed by Cascade. Every 3-4 days, the medium was removed from the samples and the hydroxyproline content measured by a standard hydroxyproline assay (Reddy, 1996).

### Hydroxyproline Content

To measure degraded collagen in the supernatant medium, the sample was lyophilized, the sample hydrolyzed in 2 M NaOH at 120°C for 20 min. After cooling, free hydroxyproline was oxidized by adding chloramine-T (Sigma) and reacting for 25 min at room temperature. Ehrlich's aldehyde reagent (Sigma) was added and allowed to react for 20 min at 65°C and followed by reading the absorbance at 550 nm on an M-5 spectrophotometer (Molecular Devices). Hydroxyproline content in the medium is an indirect measure degraded collagen and tissue remodeling potential. Cultures were incubated for up to 30 days and three samples of each treatment measured. Gels incubated without added cells were used as a control. Free peptides SILY and De13 resulted in greater collagen degradation compared to collagen alone as measured by hydroxyproline content in cell medium as shown in FIGURE 39.

### Cell Viability

Cell viability was determined using a live/dead violet viability/vitality kit (Molecular Probes. The kit contains calcein-violet stain (live cells) and aqua-fluorescent reactive dye (dead cells). Samples were washed with 1xPBS and incubated with 300 µL of dye solution for 1 hr at room temperature. To remove unbound dye, samples were rinsed with 1xPBS. Live and dead cells were counted after imaging a 2-D slice with filters 400/452 and 367/526 on an Olympus FV 1000 confocal microscope with a 20x objective. Gels were scanned for representative regions and 3 image sets were taken at equal distances into the gel for all samples.

### REFERENCE EXAMPLE 21

### Preparation of DS-Dc13

The De13 peptide sequence is SYIRIADTNITGC and its fluorescently labeled form is ZSYIRIADTNITGC, where Z designates dansylglycine. Conjugation to dermatan sulfate using the heterobifunctional crosslinker PDPH is performed as described for DS-SILY in EXAMPLE 2. As shown in FIGURE 15, the molar ratio of Dc13 to dermatan sulfate in the conjugate (DS-Dc13) was about 1.

### EXAMPLE 22

### Fluorescence Binding Assay For DS-ZSILY

The fluorescence binding assays described for DS-ZSILY was performed with peptide sequence ZSYIRIADTNITGC (ZDc13). The results appear in FIGURE 16, showing that DS-ZDc13 binds specifically to the collagen surface in a dose-dependent manner, though saturation was not achieved at the highest rate tested.

### REFERENCE EXAMPLE 23

### Fibrillogenesis Assay For DS-Dc13

A fibrillogenesis assay as described for DS-SILY, EXAMPLE 17, performed with the conjugate DS-Dc13. The results shown in FIGURE 17 indicate that the DS-Dc13 delays fibrillogenesis and decreases overall absorbance in a dose-dependent manner. Free Dc13 peptide in contrast has little effect on fibrillogenesis compared to collagen alone at the high 1:1 collagen:additive molar ratio.

### EXAMPLE 24

### Use of Cryo-SEM to Measure Fibril Diameters.

Using a modification of EXAMPLE 18 fibril diameters were measured by 8cryo-SEM. Fibril diameters from cryo-SEM images taken at 20,000x were measured using ImageJ software (NIH). At least45 fibrils were measured for each treatment. Results are presented as Avg. ± S.E. Statistical analysis was performed using DesignExpert software (StatEase) with α = 0.05. The results are shown in FIGURE 18. Decorin and synthetic peptidoglycans significantly decrease fibril diameter over collagen or collagen + dermatan sulfate. Compared to collagen alone, free peptide De13 does not affect fibril diameter while free SILY results in a decrease in fibril diameter.

### EXAMPLE 25

### Cell Culture and Gel Compaction

Human coronary artery smooth muscle cells (HCA SMC) (Cascade Biologies) were cultured in growth medium (Medium 231 supplemented with smooth muscle growth factor). Cells from passage3 were used for all experiments. Differentiation medium (Medium 231 supplemented with 1% FBS and 1x pen/strep) was used for all experiments unless otherwise noted. This medium differs from manufacturer protocol in that it does not contain heparin.

Collagen gels were prepared with each additive as described with the exception that the 1x PBS sample addition was omitted to accommodate the addition of cells in media. After incubating on ice for 30 min, HCA SMCs in differentiation medium were added to the gel solutions to a final concentration of 1 x 10⁶ cells/mL. Gels were formed in quadruplicate in 48-well non-tissue culture treated plates (Costar) for 6 hrs before adding 500 µL/well differentiation medium. Gels were freed from the well edges after 24 hrs. Medium was changed every 2-3 days and images for compaction were taken at the same time points using a Gel Doc System (Bio-Rad). The cross-sectional area of circular gels correlating to degree of compaction was determined using ImageJ software (NIH). Gels containing no cells were used as a negative control and cells in collagen gels absent additive were used as a positive control. The results are shown in FIGURE 19. By day 10 all gels had compacted to approximately 10% of the original gel area, and differences between additives were small. Gels treated with DS-Dc13 were slightly, but significantly, less compact than gels treated with decorin or collagen but compaction was statistically equivalent to that seen with DS and DS-SILY treated gels.

### EXAMPLE 26

### Measurement of Elastin

Collagen gels seeded with HCA SMCs were prepared as described in EXAMPLE 25. Differentiation medium was changed every three days and gels were cultured for 10 days. Collagen gels containing no cells were used as a control. Gels were rinsed in 1xPBS overnight to remove serum protein, and gels were tested for elastin content using the Fastin elastin assay per manufacturers protocol (Biocolor, County Atrim, U.K.). Briefly, gels were solubilized in 0.25 M oxalic acid by incubating at 100 °C for 1 hr. Elastin was precipitated and samples were then centrifuged at 11,000 x g for 10 min. The solubilized collagen supernatant was removed and the elastin pellet was stained by Fastin Dye Reagent for 90 min at room temperature. Samples were centrifuged at 11,000 x g for 10 min and unbound dye in the supernatant was removed. Dye from the elastin pellets was released by the Fastin Dye Dissociation Reagent, and 100 µL samples were transferred to a 96-well plate (Costar). Absorbance was measured at 513 nm, and elastin content was calculated from an α-elastin standard curve. The results of these assays are shown in FIGURE 20. Treatment with DS-SILY significantly increased elastin production over all samples. Treatment with DS and DS-Dc13 significantly decreased elastin production over untreated collagen. Control samples of collagen gels with no cells showed no elastin production.

### EXAMPLE 27

### Effect of Heparin or Heparin-SILY on Platelet Interaction

Collagen was immobilized on glass cover slides (18 mm) by incubating slides with collagen at 2 mg/mL in 10 mM HCl for 1 hr at 37°C. Slides were then washed with 1x PBS and stored at 4°C in 1x PBS for 24 hrs until further testing. Untreated glass cover slides were used as a negative control. Slides were placed into a 48-well non tissue-culture treated plate (Costar) with the collagen surface facing up. Heparin or Heparin-SILY were dissolved in 1x PBS to a concentration of 100 µM and incubated at 100 µL/well for 30 min at 37°C. Unbound heparin or Heparin-SILY were aspirated and the surfaces were washed with 1 mL 1x PBS. Collagen immobilized slides incubated with 1x PBS containing no additive were used as a positive control.

Whole human blood was centrifuged at 800 x g for 15 min and 100 µL of platelet-rich plasma was removed from the buffy coat layer and added to each well. After incubating for 1 hr at 37 °C, platelet-rich plasma was removed from the wells and the wells were gently washed with 1x PBS to remove unbound cells. Slides were fixed with 5% glutaraldehyde for 1 hr at room temperature, rinsed, and lyophilized before imaging. Slides were gold sputter coated for 3 min and imaged at 200x on a JEOL 840 SEM. The results are shown in FIGURE 21. This images show that treatment with the heparin-SILY conjugate affects platelet cell binding to collagen.

### EXAMPLE 28

### Cryo-SEM Measurement of Fibril Density

Collagen gels were formed in the presence of each additive at a 10:1 molar ratio, as described in EXAMPLE 15, directly on the SEM stage, processed, and imaged as described. Images at 10,000x were analyzed for fibril density calculations. Images were converted to 8-bit black and white, and threshold values for each image were determined using ImageJ software (NIH). The threshold was defined as the value where all visible fibrils are white, and all void space is black. The ratio of white to black area was calculated using MatLab software. All measurements were taken in triplicate and thresholds were determined by an observer blinded to the treatment. Images of the gels are shown in FIGURE 25 and the measured densities are shown in FIGURE 22.

### EXAMPLE 29

### Viscoelastic Characterization of Gels containing Dc13 or DS-Dc13

Collagen gels were prepared, as in EXAMPLE 15. Viscoelastic characterization was performed as described in EXAMPLE 16 on gels formed with varying ratios of collagen to additive (treatment). Treatment with dermatan sulfate or dermatan-Dc13 conjugate increase the stiffness of the resulting collagen gel over untreated collagen as shown in FIGURE 23.

### EXAMPLE 30

### Cell Proliferation and Cytotoxicity Assay

HCA SMCs, prepared as in EXAMPLE 25, were seeded at 4.8 x 10⁴ cells/mL in growth medium onto a 96-well tissue-culture black/clear bottom plate (Costar) and allowed to adhere for 4 hrs. Growth medium was aspirated and 600 µL of differentiation medium containing each additive at a concentration equivalent to the concentration within collagen gels (1.4 x 10⁻⁶ M) was added to each well. Cells were incubated for 48 hrs and were then tested for cytotoxicity and proliferation using Live-Dead and CyQuant (Invitrogen) assays, respectively, according to the manufacturer's protocol. Cells in differentiation medium containing no additive were used as control. The results are shown in FIGURE 24 indicating that none of the treatments demonstrated significant cytotoxic effects.

### EXAMPLE 31

### Inhibition of Platelet Binding and Platelet Activation to Collagen Type I

### Microplate Preparation

Type I fibrillar collagen (Chronolog, Havertown, PA) was diluted in isotonic glucose to a concentration of 20-100 µg/mL. 50 µL of collagen solution was added to each well of a high bind 96-well plate. The plate was incubated overnight at 4°C, and then rinsed 3X with 1X PBS.

Peptidoglycan was diluted in IX PBS at concentrations of 25 µM to 50 µM and 50 µL solution was added to the collagen coated wells. Controls of GAG, peptide, or PBS were also added to collagen coated wells as controls. Treatments were incubated at 37°C with shaking at 200 rpm for 30 min. Wells were then rinsed 3X with IX PBS, including a 20 min rinse with 200 rpm shaking to remove unbound treatment molecule.

### Platelet Preparation and Activation

Human whole blood was collected from healthy volunteers by venipuncture following the approved Purdue IRB protocol and with informed consent. The first 5 mL of blood was discarded as it can be contaminated with collagen and other proteins, and approximately 15 mL was then collected into citrated glass vacutainers (BD Bioscience). Blood was centrifuged in the glass tube for 20 min at 200 x g at 20°C. The top layer of the centrifuged blood, the platelet rich plasma (PRP), was used for platelet experiments. PRP (50 uL/well) was added to the microplate and allowed to incubate for 1 hr at room temperature without shaking.

After 1 hour of incubation, the PRP was removed from each well and added to a microcentrifuge tube containing 5 µL ETP (107 mM EDTA, 12 mM theophylline, and 2.8 µM prostaglandin E1) to inhibit further platelet activation. These tubes were spun at 4°C for 30 min at 1900 x g to pellet the platelets. The supernatant (platelet serum) was collected for ELISA studies to test for the presence of platelet activation markers PF-4 and Nap-2.

### Platelet Adherence

After the PRP was removed from the wells of the collagen/treatment coated plates, the wells were rinsed 3X with 0.9% NaCl for 5 min each shaking at 200 rpm. Platelet adherence was quantified colormetrically or visualized fluorescently.

### Colormetric Assay

140 µL of a sodium citrate/citric acid buffer (0.1 M, pH 5.4) containing 0.1% Triton X-100 and 1 mg/mL p-nitrophenyl phosphate was added to each well. The background absorbance was measured at 405 nm. The plate was then incubated for 40 min at room temperature with shaking at 200 rpm. The Triton X-100 creates pores in the cells, allowing p-nitrophenyl phosphate to interact with acid phosphatase in the platelets to produce p-nitrophenol. After 40 min of incubation, 100 µL of 2 M NaOH was added to each well. The pH change stops the reaction by inactivating acid phosphatase, and also transforms the p-nitrophenol to an optically active compound. The absorbance was then read at 405 nm and correlated to the number of adhered platelets. The results are shown in FIGURE 29.

### Fluorescent Assay

Adhered platelets were fixed by incubation with 4% paraformaldehyde for 10 min at room temperature. The platelets were permeabilized with 0.1% Triton X-100 for 5 min. Platelet actin was labeled by incubation with phalloidin-AlexaFluor 488 (Invitrogen) containing 1% BSA for 30 min. The wells were rinsed 3X with IX PBS, and the adhered platelets were imaged using an upright fluorescent microscope using a DAPI filter.

See FIGURES 30 to 39 for results. Platelet aggregation on untreated collagen surfaces is indicated by blurred images resulting from clumped platelets. Without being bound by theory, it is believed that clumping of platelets in the z-direction (perpendicular to the plate surface) prevents image capture in one focal plane. On treated surfaces, reduced platelet aggregation results in less clumping (fewer platelets in the z-direction), and focused images can be captured at the plate surface. These images show that treatment with the synthetic peptidoglycans reduces adhesion of platelet cells to collagen,

### Detection of Platelet Activation Markers

The supernatant (platelet serum) obtained after pelleting the platelets was used to determine released activation factors. Platelet factor 4 (PF-4) and β-thromboglobulin (Nap-2) are two proteins contained within alpha granules of platelets which are released upon platelet activation. Sandwich ELISAs were utilized in order to detect each protein. The components for both sandwich ELISAs were purchased from (R&D Systems) and the provided protocols were followed. The platelet serum samples were diluted 1:10,000 - 1:40,000 in 1% BSA in IX PBS so the values fell within a linear range. The results shown in FIGURES 27 and 28 show that treatment with synthetic peptidoglycans decreases platelet activation by collagen type I.

### EXAMPLE 32

### Inhibition of Platelet Binding and Platelet Activation to Collagen Type III and Type I

The method according to EXAMPLE 31 was used with the following modification.

### Microplate Preparation

Type I collagen (rat tail collagen, BD Biosciences) and type III collagen (Millipore) were combined on ice with NaOH, 1X PBS, and 10X PBS to physiological conditions. The total collagen concentration was 1 mg/mL with 70% type I collagen and 30% type III collagen. 30 µL of the collagen solution was pipetted into each well of a 96-well plate. The plate was incubated at 37°C in a humidified incubator for one hour, allowing a gel composed of fibrillar collagen to form in the wells. The wells were rinsed 3X with 1X PBS.

Peptidoglycan was diluted in 1X PBS at concentrations of 25vµM and 50vµL solution was added to the collagen coated wells. Controls of GAG, peptide, or PBS were also added to collagen coated wells as controls. Combinations of peptidoglycan or peptide were composed of 25 µM of each molecule in 1X PBS. Treatments were incubated at 37°C with shaking at 200 rpm for 30 min. Wells were then rinsed 3X with 1X PBS, including a 10 min rinse with 200 rpm shaking to remove unbound treatment molecule.

The results of the platelet activation inhibition measurements shown in FIGURE 39 demonstrate that the synthetic peptidoglycans inhibit platelet cell activation by a mixture of collagen Type I and Type III.

The results shown in FIGURE 40 demonstrate that the peptidoglycans inhibit platelet cell binding to collagen Type I and Type III mixtures.

### EXAMPLE 33

### Peptidoglycan Synthesis

The peptides used to synthesize the peptidoglycans described in this Example and the following Examples were synthesized by GenScript (Piscataway, New Jersey). The peptidoglycan was synthesized as described with modifications. Dermatan sulfate (DS) was oxidized by periodate oxidation in which the degree of oxidation was controlled by varying amounts of sodium meta-periodate. After oxidizing at room temperature for 2 hours protected from light, the oxidized DS was desalted into 1xPBS pH 7.2 by size exclusion chromatography using a column packed with Bio-gel P-6 (BioRad). The heterobifunctional crosslinker BMPH (Thermo Fischer Scientific) was added to oxidized DS in 30 fold molar excess to DS, and was reacted for 2 hours at room temperature protected from light. The intermediate product DS-BMPH was then purified of excess BMPH by size exclusion as described with 1xPBS pH 7.2 as running buffer. The number of BMPH crosslinkers attached to DS was calculated by the consumption of BMPH determined from the 215 nm peak area of the excess BMPH peak. A standard curve of BMPH was generated to calculate excess BMPH. The free peptide SILY was dissolved into water at a concentration of 2 mg/mL and was added in 1 molar excess to the number of attached BMPHs and was reacted for 2 hours at room temperature. The final product DS-SILYₙ was purified by size exclusion using a column packed with Sephadex G-25 medium (GE Lifesciences) with Millipore water as the running buffer. The final product was immediately frozen, lyophilized, and stored at -20°C until further testing.

A biotin labeled version of the peptidoglycan was synthesized by reacting 2 moles of SILY_{biotin} per mole of DS-BMPH for 1 hour, followed by addition of unlabeled SILY to a 1 molar excess per attached BMPH. After unlabeled SILY was added, the reaction continued for 2 hours at room temperature before purification. Biotin labeled peptidoglycan is designated as DS-SILY_{n-biotin} where n is the total number of SILY peptides per molecule. For DS-SILY_{4-biotin} only biotin labeled SILY was reacted, rather than unlabeled biotin.

### EXAMPLE 34

### Purification and Characterization of DS-BMPH

Oxidized DS was coupled to BMPH as described and purified of excess BMPH by size exclusion chromatography. As shown in Figure 41, the amount of excess BMPH is calculated by integrating the excess BMPH peak and comparing to a standard curve for BMPH. As shown in Figure 42, by varying the amount of sodium meta-periodate, the number of BMPH crosslinkers per DS chain increases linearly.

### EXAMPLE 35

### Binding Affinity of Peptidoglycan to Collagen

Fibrillar collagen (Chronolog, Havertown, PA) was coated onto the surface of a 96-well high bind plate (Costar) at a concentration of 50 µg/mL diluted in isotonic glucose. Plates were covered and incubated overnight at 4°C. Unbound collagen was removed by rinsing 3 times with 1xPBS pH 7.4. Plates were then blocked with 1% BSA for 3 hours at room temperature. Peptidoglycan was dissolved at varying concentrations in 1xPBS pH 7.4 containing 1% BSA and were immediately added to the collagen surfaces, and allowed to incubate for 15 min at room temperature. Plates were then rinsed 3 times with 1xPBS pH 7.4 containing 1% BSA. Streptavidin-HRP solution (R&D Systems, Minneapolis, MN) was then added to the plates and incubated for 20 minutes at room temperature. Unbound streptavidin was rinsed 3 times with 1xPBS pH 7.4 and 100 µL/well of color evolving solution (stabilized hydrogen peroxide and stabilized tetramethylbenzidine, R&D Systems, Minneapolis, MN) was added to each well and incubated for 20 minutes at room temperature protected from light. The color evolving reaction was stopped with 50 µL 2N sulfuric acid and absorbance was measured at 450 nm using an M5 UV Vis Spectrophotometer (Molecular Devices). Plate imperfections (540 nm) were subtracted from absorbance values.

The binding affinities of biotin labeled peptidoglycans, labeled using protocols known in the art, DS-SILY₄ and DS-SILY₁₈ were calculated by fitting the saturation binding curves and calculating the inflection point. As shown in Figure 43, DS-SILY₄ and DS-SILY₁₈ bind to fibril collagen with a K_{D} of 118 nM and 24 nM, respectively. By increasing the number of peptides per DS backbone, it is also apparent that more molecules are able to bind to the collagen surface, which is noted by the increased absorbance of DS-SILY₁₈ which does not contain more biotin label than DS-SILY₄. Consequently it is expected that DS-SILY₁₈ will show improved platelet inhibition since it can form a denser covering of the collagen surface.

### EXAMPLE 36

### Inhibition of Platelet Binding and Activation

Type I fibrillar collagen from Chronolog was diluted in isotonic glucose to a concentration of 50 µg/mL. 50 µL of collagen solution was added to each well of a high bind 96-well plate. The plate was incubated overnight at 4°C, and then rinsed 3X with 1X PBS. For microplate assays, peptidoglycan was diluted in 1X PBS at concentrations between 0.0001 µM to 100 µM and 50 µL solution was added to the collagen coated wells. DS, peptide, or 1X PBS were also added to collagen coated wells as controls. Treatments were incubated at 37°C with shaking at 200 rpm for 15 min. Wells were then rinsed of unbound treatment by removing the treatment solution, adding PBS, and shaking the wells for 24 hours. During the 24 hours, PBS solution was changed 3 times.

Human whole blood was collected from healthy volunteers by venipuncture. The first 5 mL of blood was discarded and approximately 20 mL was then collected into citrated glass vacutainers (BD Bioscience). Blood was centrifuged in the glass tube for 20 min at 200 g at 25°C. The top layer of the centrifuged blood, the platelet rich plasma (PRP), was used for platelet experiments.

PRP (50 uL/well) was added to the microplate for 1 hour at room temperature without shaking. After 1 hour of incubation, 45 µL of PRP was removed from each well and added to a microcentrifuge tube containing 5 mL ETP (107 mM EDTA, 12 mM theophylline, and 2.8 mM prostaglandin E₁) to inhibit further platelet activation. These tubes were spun at 4°C for 30 min at 2000 g to pellet the platelets. The supernatant (platelet serum) was collected for ELISA studies to test for the presence of platelet activation markers PF-4 and NAP2. Sandwich ELISAs were utilized in order to detect each protein. The components for both sandwich ELISAs were purchased from R&D Systems and the provided protocols were followed. Platelet serum was diluted 10,000 times in 1% BSA in 1X PBS for values to fall within a linear range.

Platelet activation was measured through release of platelet factor 4 (PF4) and β-thromboglobulin (NAP2). Figure 44 shows the % decrease in platelet activation by different treatments. At concentrations as high as 50 µM, DS and SILY had little to no effect on inhibiting platelet activation. Unlike individual DS or SILY, DS-SILY was able to inhibit collagen mediated platelet activation. As the number of SILY peptides per DS molecule increased from 2 to 10, the inhibition of platelet activation also increased. Since the high SILY/DS ratio is expected to provide higher binding affinity to the collagen surface due to multiple interactions, peptidoglycans with the higher SILY/DS ratios were prepared.

The number of peptides per DS molecule was further increased to 18 to create the peptidoglycan DS-SILY₁₈, and the concentration of molecule needed to inhibit platelet activation was tested. Figure 45 shows the extent of inhibition of platelet activation by DS-SILY₁₈. The data together suggest that increasing the number of peptides per DS chain further inhibits platelet binding to collagen and platelet activation. Within solubility limits, the number of peptides can be increased to achieve maximal platelet inhibition as well as to reduce diffusion over time, where a higher level of peptidoglycans on the denuded vessel wall is sustained.

### EXAMPLE 37

### Peptidoglycan Diffusion from Collagen Surface

The peptidoglycan DS-SILY_{18-biotin} was dissolved at 10 µM in 1xPBS pH 7.4 with 2% BSA and was incubated on fibrillar collagen coated plates prepared as described. The plate was incubated at 37°C on an orbital shaker and was rinsed extensively with 1xPBS pH 7.4. At various time points up to 11 days, DS-SILY_{18-biotin} was detected on the surface as described for the binding affinity studies. The curve of diffusion of peptidoglycan from the collagen surface was fitted using a hyperbolic decay.

The diffusion of the peptidoglycan DS-SILY₁₈ from a collagen surface was measured by incubating at 37°C and rinsing extensively in order to mimic blood flow in vivo. Peptidoglycan was incubated on fibrillar collagen surfaces and detected by the same methods for calculating binding affinities. As shown in Figure 46, the peptidoglycan does diffuse to some degree from the collagen surface; however, after 1 week of extensive rinsing, the equivalent of approximately 10 nM remained bound on the surface. It is estimated that complete endothelial cell regrowth occurs within 1 week of balloon injury, and thus this time frame is useful for preventing platelet binding until endothelial cells grow back and provide a permanent cover to the underlying collagen.

### EXAMPLE 38

### Endothelial Cell Proliferation

Endothelial regrowth is essential for restoring the healthy vessel and providing a permanent barrier covering the underlying collagen. Currently available drug-eluting stents for example, prevent endothelial regrowth and have consequently shown a new set of complications such as late-stent thrombosis. The peptidoglycan was tested at varying concentrations to determine if it inhibited regrowth of the endothelium. The peptidoglycan was physically bound to collagen through peptide-collagen interactions, susceptible to removal by competition binding, and thus was replaced by endothelial cells growing back over the collagen layer.

Human coronary artery endothelial cells (ECs) (Lonza, Walkersville, MD) were seeded in 96-well plates at a cell density of 1.5x10³ cells/well. Cells were allowed to adhere to the surface for 24 hours and adherent ECs were stained using cell tracker green (Invitrogen, Carlsbad, CA). Initial cell number was determined by measuring fluorescence of each well with 492 nm excitation and 517 nm emission.

DS-SILY₁₈ was solubilized in water at a concentration of 175 µM, and diluted in water 10 fold for concentrations of 175, 17.5, and 1.75 µM. The DS-SILY solution was diluted 5 fold with cell media for final concentrations of 35, 3.5, and 0.35mM. The control consisted of diluting water 5 fold with cell media. 100 µL of media with DS-SILY was added to each well and the cell number was determined 48 hours later by measuring fluorescence. The percent change in cell number in each well was calculated. As shown in Figure 47, at the highest concentration tested, there was a significant increase in cell proliferation, which suggests that the peptidoglycan promotes endothelial regrowth rather than inhibiting regrowth.

### EXAMPLE 39

### Endothelial Cell Migration

To mimic the true environment where an area of endothelial cells grown on collagen is denuded, a second test of endothelial cell migration was performed. Fibrillar collagen (Chronolog, Havertown, PA) was coated in wells of 96-well Oris Cell Migration Kit (Platypus Technologies, Madison, WI). Stoppers were inserted into the plate to block an inner circular portion of the cell. Human coronary artery endothelial cells (ECs) (Lonza, Walkersville, MD) were seeded at 5x10³ cells/well and grown to confluence in the outer portion of the well. Once confluent in the outer portion of the well, the cells were stained with cell tracker green (Invitrogen, Carlsbad, CA). The stoppers of the wells were removed, and DS-SILY₁₈ solubilized in 1X PBS was incubated on the exposed collagen surface in the inner portion of the well for 15 min at 37°C. Unbound DS-SILY was rinsed from the surface and cell media was returned to the wells. ECs were allowed to migrate from the outer portion of the wells to the inner portion for 48 hours. Fluorescence measurements of the center of each well were measured using a mask provided with the migration kit so that only the treated inner circular portion of the well was measured.

As shown in Figure 48, the same trend of increased cell number at increasing peptidoglycan concentrations was observed. Together with cell proliferation trends, the data shows that endothelial regrowth is not inhibited by peptidoglycan treatment, but that the peptidoglycan promotes regrowth.

### EXAMPLE 40

### Platelet Binding to Collagen Under Flow

To mimic physiologic conditions with flowing blood, platelet binding to collagen surfaces under flow conditions was evaluated. Flow kits were obtained from Ibidi (Munchen, Germany). Each channel was coated with fibrillar collagen (Chronolog). Excess collagen was removed from the flow channel by pushing IX PBS through the channel with a syringe. DS-SILY₁₈ was incubated in the channel at a concentration of 50 µM for 15 min at 37°C, and unbound peptidoglycan was rinsed by pushing IX PBS through the channel with a syringe. The control channel consisted of collagen not treated with peptidoglycan.

Platelet rich plasma was pushed through the channels at 2 mL/hr for 1 hour, corresponding to a shear stress of 3.55 dynes/cm² and a shear rate of 355 s⁻¹. Unbound platelets were rinsed from the channel by pushing through IX PBS. Adhered platelets were fixed in the channel with 10% formaldehyde. Platelets were permeabilized and actin filaments were stained with phalloidin-Alexa Fluor 488 (Invitrogen). Representative images of adherent platelets are shown in Figure 49, which demonstrate that significantly fewer platelets bound to the peptidoglycan treated surface in comparison to untreated collagen.

### EXAMPLE 41

### In vivo Ossabaw Pig studies

In vivo studies were performed on Ossabaw pigs in order to determine the optimal delivery method and concentration of the peptidoglycan as well as to determine preliminary efficacy of the peptidoglycan treatment. Healthy adult Ossabaw pigs underwent PCI procedures following approved protocols at Indiana University School of Medicine. In these studies, a 10 mm balloon catheter was positioned in various arteries with diameters approximately 3 to 4 mm in diameter. The balloon was inflated to 1 to 1.3 times the vessel diameter to effectively denude the vessel, and was immediately followed by a ClearwayRX delivery balloon sized to the vessel diameter.

Denuded arteries were treated by injecting through the delivery balloon between 2 and 10 mL of either saline control or various concentrations of peptidoglycan dissolved in 1xPBS pH 7.4. Angiography with contrast dye was recorded before and after each treatment to monitor balloon positioning and vessel diameters. After 14 days, pigs were sacrificed and vessels harvested for histological evaluation using H&E and Verhoff-Van Gieson staining. The pigs were heparinized during PCI procedures but were not on antiplatelet therapy at any time.

Denuded vessels treated with the sham control responded to balloon injury with significant vasospasm. Vasospasm is commonly observed in swine and is a direct consequence of platelet binding and activation on the denuded endothelium. Vasospasm was used as a measure of effective inhibition of platelet binding and inhibition of platelet activation with the peptidoglycan treatment. The severity of vasospasm corresponds to the degree of platelet deposition on the denuded endothelium. The degree of vasospasm was quantified by measuring the vessel diameter before and after balloon injury and treatment using ImageJ software, and percent occlusion calculated.

As shown in Figure 51, the peptidoglycan treatment significantly inhibited vasospasm and platelet binding to the denuded endothelium. At higher concentrations of 2.5 mg/mL or more, vasospasm is almost completely inhibited, which corresponds to *in vitro* studies in which this concentration shows maximal inhibition of platelet activation.

### EXAMPLE 42

### Histological evaluation of vessels

Histological evaluation was performed on balloon injured vessels 14 days post injury to assess intimal hyperplasia. No adverse responses were observed, and preliminary results at high peptidoglycan concentrations suggested that peptidoglycan inhibits intimal hyperplasia as shown in Figure 52.

### Delivery:

For optimal delivery of the peptidoglycan treatment, application on the denuded endothelium should occur immediately following balloon injury. The peptidoglycan can prevent platelets from binding to the denuded endothelium. A system in which a single balloon capable of expanding the vessel as well as delivering the peptidoglycan treatment can be used, however effective platelet inhibition is achieved under current delivery protocols.

### EXAMPLE 43

### Immunohistochemistry

Fresh carotid arteries were harvested from pigs and placed in cold 1x PBS, and tested within 5 hours of harvest. Arteries were cut open and denuded with a rubber policeman and were then cut into approximately 4 mm segments and placed into a 96-well plate. Biotin labeled peptidoglycan DS-SILY18-biotin was incubated at 10µM dissolved in 1x PBS pH 7.4 for 15 min at room temperature. Control arteries were incubated with 1x PBS pH 7.4. Arteries were snap frozen in liquid nitrogen, cut into 7µm sections and air dried for 45 min, then stored at -20°C until staining. Tissue was fixed in ice cold acetone, air dried and washed with DI water. Sections were incubated with streptavidin-HRP for 30 min, washed with DI water, incubated with DAB for 10 min, rinsed and stained with hematoxylin for 5 min. Brightfield images were taken at 10x.

As shown in Figure 53, denuded arteries incubated with labeled peptidoglycan stained positive at the denuded surface in contrast to control arteries which did not take up the stain. The peptidoglycan uniformly bound largely at the surface, which has a higher concentration of collagen, rather than deeper into the tissue.

### EXAMPLE 44

### Inhibition of whole blood binding to collagen under flow

Flow kits were obtained from Ibidi (Martinsried, Germany). Each channel was coated with fibrillar collagen as described for static microplate studies. Excess collagen was removed from the flow channel by extensive rinsing with IX PBS through the channel. DS-SILY₁₈ was incubated in the channel at a concentration of 50 µM for 15 min at 37°C, and unbound peptidoglycan was rinsed with 1XPBS. Control channels consisted of collagen not treated with peptidoglycan.

Whole blood was pushed through the flow channels by a syringe pump at a flow rate of 5.6 mL/hr, corresponding to a physiologically relevant shear rate of 1000 s⁻¹ (Badimon L, Badimon JJ, Turitto VT, Vallabhajosula S, Fuster V. Platelet thrombus formation on collagen type I - a model of deep vessel injury - influence of blood rheology, von Willebrand Factor, and blood-coagulation. Circulation 1988; 78(6):1431-42). After 5 min of flow, IX PBS pH 7.4 was pushed through at the same flow rate for 10 min to wash unbound cells. Brightfield images (n = 3) were taken of each flow channel with a 10X objective. Images were thresholded and quantified for cellular coverage using ImageJ (NIH, Bethesda, MD) and MatLab (Mathworks, Natick, MA) respectively. Control channels were assumed to have complete cellular coverage. The results in Figure 54 show that there was much less blood cell binding to collagen when collagen was treated with DS-SILY₁₈.

## Claims

1. A collagen binding peptidoglycan of formula (PₙL)ₓG,
wherein n is 1 to 7,
x is 1 to 10,
P is a synthetic peptide of up to 40 amino acids comprising a sequence of RRANAALKAGELYKSILY,
L is a linker, and
G is glycan,
for use in a method for treating:
i) a vascular injury; or
ii) intimal hyperplasia.

2. The collagen binding peptidoglycan for use according to claim 1, wherein the compound is administered to a patient prior to, during, or after vascular intervention.

3. The collagen binding peptidoglycan for use according to claim 2, wherein the vascular intervention is angioplasty, particularly balloon angioplasty.

4. The collagen binding peptidoglycan for use according to claim 1 or 2, wherein the collagen binding peptidoglycan is administered intravascularly.

5. The collagen binding peptidoglycan for use according to claim 1, wherein the linker has a molecular weight of 20 to 500 Daltons.

6. The collagen binding peptidoglycan for use according to claim 1, wherein the glycan is a glycosaminoglycan.

7. The collagen binding peptidoglycan for use according to claim 1, wherein the glycan is alginate, agarose, dextran, chondroitin, dermatan, dermatan sulfate, heparan, heparin, keratin, or hyaluronan.

8. The collagen binding peptidoglycan for use according to claim 1, wherein the glycan is dermatan sulfate.

9. The collagen binding peptidoglycan for use according to claim 1, wherein the glycan is heparin.

## Patentansprüche

1. Kollagenbindendes Peptidoglykan der Formel (PₙL)ₓG, wobei n 1 bis 7 ist,
x 1 bis 10 ist,
P ein synthetisches Peptid von bis zu 40 Aminosäuren, umfassend eine Sequenz von RRANAALKAGELYKSILY, ist,
L ein Linker ist, und
G Glykan ist,
zur Verwendung in einem Verfahren zur Behandlung von:
i) einer Gefäßverletzung; oder
ii) Intimahyperplasie.

2. Kollagenbindendes Peptidoglykan zur Verwendung nach Anspruch 1, wobei die Verbindung einem Patienten vor, während, oder nach einem Gefäßeingriff verabreicht wird.

3. Kollagenbindendes Peptidoglykan zur Verwendung nach Anspruch 2, wobei es sich bei dem Gefäßeingriff um Angioplastie, insbesondere Ballonangioplastie, handelt.

4. Kollagenbindendes Peptidoglykan zur Verwendung nach Anspruch 1 oder 2, wobei das kollagenbindende Peptidoglykan intravaskulär verabreicht wird.

5. Kollagenbindendes Peptidoglykan zur Verwendung nach Anspruch 1, wobei der Linker ein Molekulargewicht von 20 bis 500 Dalton aufweist.

6. Kollagenbindendes Peptidoglykan zur Verwendung nach Anspruch 1, wobei das Glykan ein Glykosaminoglykan ist.

7. Kollagenbindendes Peptidoglykan zur Verwendung nach Anspruch 1, wobei es sich bei dem Glykan um Alginat, Agarose, Dextran, Chondroitin, Dermatan, Dermatansulfat, Heparan, Heparin, Keratin, oder Hyaluronan handelt.

8. Kollagenbindendes Peptidoglykan zur Verwendung nach Anspruch 1, wobei es sich bei dem Glykan um Dermatansulfat handelt.

9. Kollagenbindendes Peptidoglykan zur Verwendung nach Anspruch 1, wobei es sich bei dem Glykan um Heparin handelt.

## Revendications

1. Peptidoglycane se liant au collagène de formule
(PₙL)ₓG,
dans lequel
n vaut 1 à 7,
x vaut 1 à 10,
P est un peptide synthétique comprenant jusqu'à 40 acides aminés comprenant une séquence RRANAALKAGELYKSILY,
L est un lieur et
G est un glycane,
destiné à être utilisé dans une méthode pour le traitement :
i) d'une lésion vasculaire ; ou
ii) d'une hyperplasie intimale.

2. Peptidoglycane se liant au collagène destiné à être utilisé selon la revendication 1, le composé étant administré à un patient avant, pendant ou après une intervention vasculaire.

3. Peptidoglycane se liant au collagène destiné à être utilisé selon la revendication 2, l'intervention vasculaire étant une angioplastie, en particulier une angioplastie par ballonnet.

4. Peptidoglycane se liant au collagène destiné à être utilisé selon la revendication 1 ou 2, le peptidoglycane se liant au collagène étant administré par voie intravasculaire.

5. Peptidoglycane se liant au collagène destiné à être utilisé selon la revendication 1, dans lequel le lieur a une masse moléculaire de 20 à 500 daltons.

6. Peptidoglycane se liant au collagène destiné à être utilisé selon la revendication 1, dans lequel le glycane est un glycosaminoglycane.

7. Peptidoglycane se liant au collagène destiné à être utilisé selon la revendication 1, dans lequel le glycane est l'alginate, l'agarose, le dextrane, la chondroïtine, le dermatane, le dermatane sulfate, l'héparane, l'héparine, la kératine ou le hyaluronane.

8. Peptidoglycane se liant au collagène destiné à être utilisé selon la revendication 1, dans lequel le glycane est le dermatane sulfate.

9. Peptidoglycane se liant au collagène destiné à être utilisé selon la revendication 1, dans lequel le glycane est l'héparine.


### SEQUENCE LISTING

<110> PURDUE RESEARCH FOUNDATION
<120> COLLAGEN-BINDING SYNTHETIC PEPTIDOGLYCANS FOR USE IN VASCULAR INTERVENTION
<130> EP85664HVSZpau
<140> EP11798931.9
   <141> 2011-06-23
<150> PCT/US2011/041654
   <151> 2011-06-23
<150> 61/357,912
   <151> 2010-06-23
<160> 30
<170> PatentIn version 3.5
<210> 1
   <211> 20
   <212> PRT
   <213> Artificial Sequence
<220>
   <221> source
   <223> /note="Description of Artificial Sequence: Synthetic peptide"
<400> 1
   Arg Arg Ala Asn Ala Ala Leu Lys Ala Gly Glu Leu Tyr Lys Ser Ile
   1               5                   10                  15
   Leu Tyr Gly Cys
               20
<210> 2
   <211> 11
   <212> PRT
   <213> Artificial Sequence
<220>
   <221> source
   <223> /note="Description of Artificial Sequence: Synthetic peptide"
<400> 2
   Arg Leu Asp Gly Asn Glu Ile Lys Arg Gly Cys
   1               5                   10
<210> 3
   <211> 15
   <212> PRT
   <213> Artificial Sequence
<220>
   <221> source
   <223> /note="Description of Artificial Sequence: Synthetic peptide"
<400> 3
   Ala His Glu Glu Ile Ser Thr Thr Asn Glu Gly Val Met Gly Cys
   1               5                   10                  15
<210> 4
   <211> 30
   <212> PRT
   <213> Artificial Sequence
<220>
   <221> source
   <223> /note="Description of Artificial Sequence: Synthetic polypeptide"
<400> 4
   Asn Gly Val Phe Lys Tyr Arg Pro Arg Tyr Phe Leu Tyr Lys His Ala
   1               5                   10                  15
   Tyr Phe Tyr Pro Pro Leu Lys Arg Phe Pro Val Gln Gly Cys
               20                  25                  30
<210> 5
   <211> 10
   <212> PRT
   <213> Artificial Sequence
<220>
   <221> source
   <223> /note="Description of Artificial Sequence: Synthetic peptide"
<400> 5
   Cys Gln Asp Ser Glu Thr Arg Thr Phe Tyr
   1               5                   10
<210> 6
   <211> 9
   <212> PRT
   <213> Artificial Sequence
<220>
   <221> source
   <223> /note="Description of Artificial Sequence: Synthetic peptide"
<400> 6
   Thr Lys Lys Thr Leu Arg Thr Gly Cys
   1               5
<210> 7
   <211> 30
   <212> PRT
   <213> Artificial Sequence
<220>
   <221> source
   <223> /note="Description of Artificial Sequence: Synthetic polypeptide"
<400> 7
   Gly Leu Arg Ser Lys Ser Lys Lys Phe Arg Arg Pro Asp Ile Gln Tyr
   1               5                   10                  15
   Pro Asp Ala Thr Asp Glu Asp Ile Thr Ser His Met Gly Cys
               20                  25                  30
<210> 8
   <211> 9
   <212> PRT
   <213> Artificial Sequence
<220>
   <221> source
   <223> /note="Description of Artificial Sequence: Synthetic peptide"
<400> 8
   Ser Gln Asn Pro Val Gln Pro Gly Cys
   1               5
<210> 9
   <211> 13
   <212> PRT
   <213> Artificial Sequence
<220>
   <221> source
   <223> /note="Description of Artificial Sequence: Synthetic peptide"
<400> 9
   Ser Tyr Ile Arg Ile Ala Asp Thr Asn Ile Thr Gly Cys
   1 5 10
<210> 10
   <211> 11
   <212> PRT
   <213> Artificial Sequence
<220>
   <221> source
   <223> /note="Description of Artificial Sequence: Synthetic peptide"
<400> 10
   Ser Tyr Ile Arg Ile Ala Asp Thr Asn Ile Thr
   1 5 10
<210> 11
   <211> 8
   <212> PRT
   <213> Artificial Sequence
<220>
   <221> source
   <223> /note="Description of Artificial Sequence: Synthetic peptide"
<400> 11
   Lys Glu Leu Asn Leu Val Tyr Thr
   1 5
<210> 12
   <211> 10
   <212> PRT
   <213> Artificial Sequence
<220>
   <221> source
   <223> /note="Description of Artificial Sequence: Synthetic peptide"
<400> 12
   Lys Glu Leu Asn Leu Val Tyr Thr Gly Cys
   1 5 10
<210> 13
   <211> 11
   <212> PRT
   <213> Artificial Sequence
<220>
   <221> source
   <223> /note="Description of Artificial Sequence: Synthetic peptide"
<400> 13
   Gly Glu Leu Tyr Lys Ser Ile Leu Tyr Gly Cys
   1               5                   10
<210> 14
   <211> 12
   <212> PRT
   <213> Artificial Sequence
<220>
   <221> source
   <223> /note="Description of Artificial Sequence: Synthetic peptide"
<400> 14
   Gly Ser Ile Thr Thr Ile Asp Val Pro Trp Asn Val
   1               5                   10
<210> 15
   <211> 12
   <212> PRT
   <213> Artificial Sequence
<220>
   <221> source
   <223> /note="Description of Artificial Sequence: Synthetic peptide"
<400> 15
   Gly Cys Gly Gly Glu Leu Tyr Lys Ser Ile Leu Tyr
   1               5                   10
<210> 16
   <211> 14
   <212> PRT
   <213> Artificial Sequence
<220>
   <221> source
   <223> /note="Description of Artificial Sequence: Synthetic peptide"
<400> 16
   Gly Ser Ile Thr Thr Ile Asp Val Pro Trp Asn Val Gly Cys
   1               5                   10
<210> 17
   <211> 18
   <212> PRT
   <213> Artificial Sequence
<220>
   <221> source
   <223> /note="Description of Artificial Sequence: Synthetic peptide"
<400> 17
   Arg Arg Ala Asn Ala Ala Leu Lys Ala Gly Glu Leu Tyr Lys Ser Ile
   1               5                   10                  15
   Leu Tyr
<210> 18
   <211> 9
   <212> PRT
   <213> Artificial Sequence
<220>
   <221> source
   <223> /note="Description of Artificial Sequence: Synthetic peptide"
<400> 18
   Arg Leu Asp Gly Asn Glu Ile Lys Arg
   1               5
<210> 19
   <211> 13
   <212> PRT
   <213> Artificial Sequence
<220>
   <221> source
   <223> /note="Description of Artificial Sequence: Synthetic peptide"
<400> 19
   Ala His Glu Glu Ile Ser Thr Thr Asn Glu Gly Val Met
   1               5                   10
<210> 20
   <211> 28
   <212> PRT
   <213> Artificial Sequence
<220>
   <221> source
   <223> /note="Description of Artificial Sequence: Synthetic peptide"
<400> 20
   Asn Gly Val Phe Lys Tyr Arg Pro Arg Tyr Phe Leu Tyr Lys His Ala
   1               5                   10                  15
   Tyr Phe Tyr Pro Pro Leu Lys Arg Phe Pro Val Gln
               20                  25
<210> 21
   <211> 7
   <212> PRT
   <213> Artificial Sequence
<220>
   <221> source
   <223> /note="Description of Artificial Sequence: Synthetic peptide"
<400> 21
   Thr Lys Lys Thr Leu Arg Thr
   1               5
<210> 22
   <211> 28
   <212> PRT
   <213> Artificial Sequence
<220>
   <221> source
   <223> /note="Description of Artificial Sequence: Synthetic peptide"
<400> 22
   Gly Leu Arg Ser Lys Ser Lys Lys Phe Arg Arg Pro Asp Ile Gln Tyr
   1               5                   10                  15
   Pro Asp Ala Thr Asp Glu Asp Ile Thr Ser His Met
               20                  25
<210> 23
   <211> 7
   <212> PRT
   <213> Artificial Sequence
<220>
   <221> source
   <223> /note="Description of Artificial Sequence: Synthetic peptide"
<400> 23
   Ser Gln Asn Pro Val Gln Pro
   1               5
<210> 24
   <211> 11
   <212> PRT
   <213> Artificial Sequence
<220>
   <221> source
   <223> /note="Description of Artificial Sequence: Synthetic peptide"
<400> 24
   Ser Tyr Ile Arg Ile Ala Asp Thr Asn Ile Thr
   1               5                   10
<210> 25
   <211> 9
   <212> PRT
   <213> Artificial Sequence
<220>
   <221> source
   <223> /note="Description of Artificial Sequence: Synthetic peptide"
<400> 25
   Gly Glu Leu Tyr Lys Ser Ile Leu Tyr
   1               5
<210> 26
   <211> 12
   <212> PRT
   <213> Artificial Sequence
<220>
   <221> source
   <223> /note="Description of Artificial Sequence: Synthetic peptide"
<400> 26
   Cys Gln Asp Ser Glu Thr Arg Thr Phe Tyr Gly Cys
   1               5                   10
<210> 27
   <211> 14
   <212> PRT
   <213> Artificial Sequence
<220>
   <221> source
   <223> /note="Description of Artificial Sequence: Synthetic peptide"
<400> 27
   Gly Cys Gly Gly Glu Leu Tyr Lys Ser Ile Leu Tyr Gly Cys
   1               5                   10
<210> 28
   <211> 21
   <212> PRT
   <213> Artificial Sequence
<220>
   <221> source
   <223> /note="Description of Artificial Sequence: Synthetic peptide"
<220>
   <221> MOD_RES
   <222> (1)..(1)
   <223> Dansyl-Gly
<400> 28
   Gly Arg Arg Ala Asn Ala Ala Leu Lys Ala Gly Glu Leu Tyr Lys Ser
   1 5 10 15
   Ile Leu Tyr Gly Cys
   20
<210> 29
   <211> 12
   <212> PRT
   <213> Artificial Sequence
<220>
   <221> source
   <223> /note="Description of Artificial Sequence: Synthetic peptide"
<220>
   <221> MOD_RES
   <222> (1)..(1)
   <223> Dansyl-Gly
<400> 29
   Gly Ser Tyr Ile Arg Ile Ala Asp Thr Asn Ile Thr
   1 5 10
<210> 30
   <211> 14
   <212> PRT
   <213> Artificial Sequence
<220>
   <221> source
   <223> /note="Description of Artificial Sequence: Synthetic peptide"
<220>
   <221> MOD_RES
   <222> (1)..(1)
   <223> Dansyl-Gly
<400> 30
   Gly Ser Tyr Ile Arg Ile Ala Asp Thr Asn Ile Thr Gly Cys
   1 5 10